(19) Europäisches Patentamt
European Patent Office
Office européen des brevets

(11) **EP 4 647 742 A1**

(12) **EUROPEAN PATENT APPLICATION**
published in accordance with Art. 153(4) EPC

(43) Date of publication:
**12.11.2025 Bulletin 2025/46**

(21) Application number: **24738578.4**

(22) Date of filing: **05.01.2024**

(51) International Patent Classification (IPC):
***G01N 21/64*** (2006.01)

(52) Cooperative Patent Classification (CPC):
**C12M 1/00; C12M 1/34; G01N 21/64; G02B 21/06; G02B 21/10; G02B 21/16**

(86) International application number:
**PCT/CN2024/070947**

(87) International publication number:
**WO 2024/146647 (11.07.2024 Gazette 2024/28)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **06.01.2023 CN 202320043163 U**
**25.06.2023 CN 202310756783**

(71) Applicant: **GeneMind Biosciences Company Limited**
**Shenzhen, Guangdong 518023 (CN)**

(72) Inventors:
• **BO, En**
**Shenzhen, Guangdong 518023 (CN)**

• **SONG, Xiaokang**
**Shenzhen, Guangdong 518023 (CN)**
• **ZHAN, Yongbo**
**Shenzhen, Guangdong 518023 (CN)**
• **LI, Yang**
**Shenzhen, Guangdong 518023 (CN)**
• **WANG, Penglei**
**Shenzhen, Guangdong 518023 (CN)**
• **WU, Ping**
**Shenzhen, Guangdong 518023 (CN)**
• **JIANG, Zefei**
**Shenzhen, Guangdong 518023 (CN)**
• **WANG, Guangming**
**Shenzhen, Guangdong 518023 (CN)**

(74) Representative: **Schiweck Weinzierl Koch Patentanwälte Partnerschaft mbB**
**Ganghoferstraße 68 B**
**80339 München (DE)**

(54) **OPTICAL SYSTEM AND GENE SEQUENCING DEVICE**

(57) The present application discloses an optical system and a gene sequencing device. The optical system comprises an illumination light path assembly, an imaging light path assembly, and a driving component. The illumination light path assembly comprises a light source and a light modulation component, and the light modulation component is used for transmitting and modulating illumination light generated by the light source to form an illumination light field. The imaging light path assembly comprises an objective lens and an optical imaging assembly; the objective lens is disposed on a light path of the illumination light field; the objective lens is used for projecting the illumination light field onto a sample and receiving an optical signal generated from the sample; the optical imaging assembly is used for receiving the optical signal and forming an image. The driving component is used for driving the light modulation component and the sample to move relative to the objective lens, so as to update the pattern of the illumination light field located on the sample. By using the optical system of the present solution, the imaging efficiency of the optical system is effectively improved, and the complexity of the system and production and manufacturing costs are reduced.

FIG. 1

EP 4 647 742 A1

**Description**

TECHNICAL FIELD

The present application relates to the field of optical imaging technology, and in particular, to an optical system and a gene sequencing device.

BACKGROUND

The super-resolution (SR) imaging is an imaging technique that can improve the image resolution by overcoming the optical diffraction limit. After nearly 30 years of development, the super-resolution imaging technique has been oriented to techniques including the stochastic optical reconstruction microscopy, photoactivated localization microscopy, stimulated emission depletion microscopy, and structured illumination microscopy. The resolutions of the first three super-resolution imaging techniques can be improved to achieve a super-resolution of less than 100 nm. However, they may need to acquire a lot of original images or to perform point-by-point scanning to reconstruct a super-resolution image, thus featuring a reduced overall super-resolution imaging throughput. The super-resolution imaging technique based on structured illumination microscopy has attracted a great many attentions due to the even higher increase in the resolution. Currently, optical elements used in the super-resolution imaging technique based on structured illumination microscopy include digital micro-mirror devices (DMDs), liquid crystal spatial light modulators (LC-SLMs), and the like. In an optical system based on DMD optical elements, changes in the direction and phase shift of structured light fringes are achieved in a high-speed electronic control manner with negligible impact on the imaging speed. However, the small area arrays of commercially available DMDs may lead to a super-resolution field of view smaller than the field of view of the objective lens and thus the loss of imaging throughput. An optical system based on LC-SLM optical elements has the advantage of large area arrays and no limitations to the super-resolution imaging field of view, with the size of the super-resolution field of view dependent on the field of view of the objective lens. However, since the optical system is based on electrically controlled liquid crystal switches, both the direction and the phase shift of structured light fringes change slowly, thereby limiting the imaging speed.

Therefore, it is particularly important to find an optical system that can keep a balance between a desirable size of the super-resolution field of view and the imaging efficiency.

SUMMARY

The present application provides an optical system and a gene sequencing device.

The optical system according to embodiments of the present application includes an illumination optical path assembly, an imaging optical path assembly, and a driving component. The illumination optical path assembly includes a light source and a light modulation component. The light modulation component is configured for transmitting and modulating an illumination light generated by the light source to form an illumination light field. The imaging optical path assembly includes an objective lens and an optical imaging assembly. The objective lens is arranged on an optical path of the illumination light field and configured for projecting the illumination light field onto a sample and receiving an optical signal generated by the sample. The optical imaging assembly is configured for receiving the optical signal and forming an image. The driving component is configured for driving the light modulation component and/or the sample to move relative to the objective lens, so as to update a pattern of the illumination light field located on the sample. The implementation of the embodiments of the present application has the following beneficial effects:

By using the optical system of the present disclosure, the light modulation component modulates the illumination light to form patterned illumination light. Therefore, the optical system has no limitations on the super-resolution field of view and can accurately update the pattern of the illumination light field on the sample. In addition, the driving component drives the light modulation component and/or the sample to move relative to the objective lens, so as to update the pattern of the illumination light field on the sample, thereby effectively improving the imaging efficiency of the optical system. The optical system according to the solution features high robustness in super-resolution imaging. Moreover, the optical system features a simple structure, and the optical system in which the driving component drives the light modulation component and/or the sample to move relative to the objective lens is beneficial to reducing the complexity of the system and the manufacturing costs.

The gene sequencing device according to the embodiments of the present application includes an optical system. The sequencing device according to embodiments of the present application includes, but is not limited to, devices having sequencing functionality such as gene sequencing devices and nucleic acid sequencing devices, while other systems or devices manufactured as per the same principle are also applicable to the embodiments of the present application.

The additional aspects and advantages of the present application will be partially set forth in the following description, and will partially become apparent from the following description or be appreciated by practice of the present application.

BRIEF DESCRIPTION OF THE DRAWINGS

In order to more clearly illustrate the technical solutions in the examples of the present application or the prior art, the drawings required for use in the description of the examples or the prior art will be briefly described below. It is obvious that the drawings in the description below are only some examples of the present application, and other drawings can be derived from these drawings by those of ordinary skill in the art without creative efforts.

Among the drawings:

FIG. 1 is a structural block diagram of an optical system according to the embodiments of the present application;
FIG. 2 is a schematic view of an optical system according to the embodiments of the present application;
FIG. 3 is a schematic view of an optical system according to the embodiments of the present application;
FIG. 4 is a schematic view of an optical system according to the embodiments of the present application;
FIG. 5 is a schematic view of an optical system according to the embodiments of the present application;
FIG. 6 is a schematic view of an optical system according to the embodiments of the present application;
FIG. 7 is a schematic view of an optical system according to the embodiments of the present application;
FIG. 8 is a schematic view of an optical system according to the embodiments of the present application;
FIG. 9 is a schematic view of an optical system according to the embodiments of the present application;
FIG. 10 is a structural schematic view of a tube lens according to the embodiments of the present application;
FIG. 11 is MTF curves of a tube lens according to the embodiments of the present application;
FIG. 12 is a spot diagram of a tube lens according to the embodiments of the present application;
FIG. 13 is a Seidel diagram of a tube lens according to the embodiments of the present application;
FIG. 14 is a focal shift curve of a tube lens according to the embodiments of the present application;
FIG. 15 is a top view of a light modulation component according to the embodiments of the present application;
FIG. 16 is a schematic view of the optimization of an illumination light field according to the embodiments of the present application: (a) a micro-lens array; (b) an illumination light field generated by modulation of the micro-lens array; (c) random illumination light intensity of a certain row (as shown by the dashed line in panel b); (d) a binarized light homogenizer; (e) an illumination light field generated by the modulation of the binarized light homogenizer; and (f) random illumination light intensity of a certain row (as shown by the dashed line in panel e).
FIG. 17 is a structural schematic view of a first driving component according to the embodiments of the present application from one angle of view;
FIG. 18 is a structural schematic view of the first driving component according to the embodiments of the present application from another angle of view;
FIG. 19 is a structural schematic view of a second driving component according to the embodiments of the present application.

Description of reference numerals: 100, imaging optical path assembly; 10, first plane; 20, second plane; 30, third plane; 110, objective lens; 111, optical axis; 120, optical imaging assembly; 121, first camera; 122, second camera; 123, third camera; 124, fourth camera; 130, splitter mechanism; 131, mirror; 132, illumination dichroic mirror; 133, fourth dichroic mirror; 140, first splitter mechanism; 141, first dichroic mirror; 142, second dichroic mirror; 143, first mirror; 150, second splitter mechanism; 151, third dichroic mirror; 160, third splitter mechanism; 161, second mirror; 170, fourth splitter mechanism; 171, third mirror; 180, tube lens; 181, first lens; 182, second lens; 183, third lens; 184, fourth lens; S1, object-side surface of first lens; S2, image-side surface of first lens; S3, object-side surface of second lens; S4, image-side surface of second lens; S5, object-side surface of third lens; S6, image-side surface of third lens; S7, object-side surface of fourth lens; S8, image-side surface of fourth lens; 190, filter; 200, illumination optical path assembly; 210, light source; 211, first light beam; 212, second light beam; 213, third light beam; 214, fourth light beam; 215, first mixed light beam; 216, second mixed light beam; 220, light modulation component; 221, transmissive region; 222, opaque region; 230, illumination lens set; 240, illumination mirror; 300, driving component; 301, first driving component; 302, second driving component; 310, driving structure; 320, mounting member; 321, extension plate; Y, first direction; X, second direction; Z, third direction; 311, first sliding assembly; 3111, first fixed member; 3112, first sliding member; 3113, first driving member; 3114, first limiting plate; 3115, first motor; 3116, first driving shaft; 312, second sliding assembly; 3121, second fixed member; 3122, second sliding member; 3123, second driving member; 3124, second limiting plate; 3125, second motor; 3126, second driving shaft; 313, third sliding assembly; 3131, third fixed member; 3132, third sliding member; 3133, third driving member; 3134, third limiting plate; 330, carrying platform; 340, two-dimensional driving assembly; 400, focusing assembly; 410, second light source; 420, focusing sensor; 1000, optical system; M, MTF curve of diffraction limit.

DETAILED DESCRIPTION

The technical solutions in the examples of the present application will be described below clearly and comprehensively in conjunction with the drawings in the examples of the present application. It is obvious that the described examples are part of the examples of the present application, but not all of them. On the basis of the examples of the present application, all other examples obtained by those of ordinary skill in the art without creative efforts shall fall within the claimed scope of the present application.

In the description of the present application, it should be understood that orientational or positional relationships indicated by terms such as "central", "longitudinal", "transverse", "length", "width", "thickness", "upper", "lower", "front", "rear", "left", "right", "vertical", "horizontal", "top", "bottom", "inner", "outer", "clockwise", or "counterclockwise", are those shown on the basis of the drawings, and are merely intended to facilitate and simplify the description rather than indicate or imply that the indicated apparatus or element must have a specific orientation and be configured and operated according to the specific orientation. Such relationships should not be construed as limiting the present application. In addition, the terms "first" and "second" are used herein for descriptive purposes only and should not be construed as indicating or implying relative importance or implicitly indicating the number of technical features described. Therefore, features defined with "first" and "second" may explicitly or implicitly include one or more of the features. In the description of the present application, unless otherwise specifically defined, "a plurality of" means two or more than two.

In the description of the present application, it should be noted that unless otherwise clearly specified and defined, the terms "mount", "link", and "connect" should be interpreted in their broad sense. For example, the connection may be a fixed connection, detachable connection, or integral connection; a mechanical connection, electric connection, or communicative connection; or a direct connection, indirect connection through an intermediate, internal communication of two elements, or interaction between two elements. For those of ordinary skill in the art, the specific meanings of the aforementioned terms in the present application can be interpreted according to specific conditions.

In the present application, unless otherwise clearly specified and defined, a first feature being "above" or "below" a second feature may include that the first and second features are in direct contact and that the first and second features are not in direct contact but are in contact via an additional feature therebetween. Moreover, a first feature being "on", "over", and "above" a second feature includes that the first feature is right above or obliquely above the second feature, or simply means that the first feature is at a vertically higher position than the second feature. A first feature being "under", "beneath", and "below" a second feature includes that the first feature is right below or obliquely below the second feature, or simply means that the first feature is at a vertically lower position than the second feature.

In the embodiments of the present application, the term "sequencing" may also be referred to as "nucleic acid sequencing" or "gene sequencing". The three are used interchangeably and refer to the determination of the type and order of bases or nucleotides (including nucleotide analogs) in a nucleic acid molecule. The sequencing involves the process of binding nucleotides to a template and collecting the corresponding signals emitted by the nucleotides (including analogs). The sequencing includes sequencing by synthesis (SBS) and/or sequencing by ligation (SBL), DNA sequencing and/or RNA sequencing, and long fragment sequencing and/or short fragment sequencing (the long fragment and short fragment are defined relatively; e.g., nucleic acid molecules longer than 1 Kb, 2 Kb, 5 Kb, or 10 Kb may be referred to as long fragments, and nucleic acid molecules shorter than 1 Kb or 800 bp may be referred to as short fragments).

The sequencing generally involves multiple cycles of process to achieve the determination of the types and sequence of multiple bases or nucleotides on the nucleic acid template. The examples of the present application refer to each cycle of the "process to achieve the determination of the types and sequence of multiple bases or nucleotides on the nucleic acid template" as one "cycle of sequencing". The "cycle of sequencing", also known as "sequencing cycle", may be defined as the completion of one base extension of the four types of nucleotides/bases; in other words, one "cycle of sequencing" may be defined as the determination of the base or nucleotide type at any given position on the template. For sequencing platforms that achieve sequencing on the basis of polymerization or ligation reactions, one cycle of sequencing includes the process of binding four types of nucleotides (including nucleotide analogs) to the nucleic acid template at a time according to the base complementary rule and acquiring the corresponding signals emitted. For platforms that achieve sequencing on the basis of the polymerization reaction, a reaction system includes reaction substrate nucleotides, polymerase, and a nucleic acid template. A sequence fragment (a sequencing primer) binds to the nucleic acid template, and on the basis of the base pairing rules and the principle of polymerization reaction, the added reaction substrate nucleotides are linked to the sequencing primer under the catalysis of the polymerase to achieve the binding of the nucleotide to a specific position on the nucleic acid template. Generally, one cycle of sequencing may include one or more base extensions (repeats). For example, four types of nucleotides are sequentially added to the reaction system to each perform base extensions and corresponding acquisition of reaction signals, and one cycle of sequencing includes four base extensions; for another example, four types of nucleotides are added into the reaction system in any combinations (such as in pairs or in one-three combinations), the two combinations each perform base extensions and corresponding acquisition of reaction signals, and one cycle of sequencing includes two base extensions; for yet another example, four types of nucleotides are added simultaneously to the reaction system for base extension and reaction signal acquisition, and one cycle of sequencing includes one base extension.

The term "nucleic acid molecule" refers to polymeric forms of nucleotides of any length and may include ribonucleotides or analogs thereof, deoxyribonucleotides or analogs thereof, and mixtures of the above nucleotides or analogs thereof. The nucleic acid molecule may refer to a single-stranded polynucleotide or a double-stranded polynucleotide. Nucleotides in a nucleic acid molecule may include naturally occurring nucleotides and functionally alternative analogs thereof. Examples of analogs can hybridize to nucleic acids in a sequence-specific manner, or can be used as templates for the replication of particular nucleotide sequences. Naturally occurring nucleotides generally have a backbone containing a phosphodiester bond. Analog structures may have alternative backbone linkages including any types known in the art. Naturally occurring nucleotides generally have deoxyribose (e.g., found in DNA) or ribose (e.g., found in RNA). Analog structures may have alternative sugar moieties including any types known in the art. Nucleotides may contain natural bases or non-natural bases. Bases in natural DNA may include one or more of adenine, thymine, cytosine, and/or guanine, and bases in natural RNA may include one or more of adenine, uracil, cytosine, and/or guanine. Any non-natural base or base analog may also be used in a nucleotide, such as a locked nucleic acid (LNA) and a bridged nucleic acid (BNA).

The term "nucleic acid template" refers to a master nucleic acid molecule or master nucleic acid molecule fragment that binds to nucleotides or nucleotide analogs by multiple successive base extension cycles. The nucleic acid template may be the entire sequence of the nucleic acid molecule or a partial fragment of the nucleic acid molecule. The "nucleic acid template" may be a nucleic acid fragment used as a template in extension reactions in which sequencing by synthesis is performed; since the base added to the extension reactions and the sequencing template satisfy the base pairing rules, the sequence of the sequencing template can be determined by determining the type of the base added to each extension cycle. The sequence of the nucleic acid template herein may generally include, but is not limited to, at least one of the sequence of the target molecule, the UMI sequence, and the sample tag sequence, and may sometimes also be referred to herein as an "insert" or "target molecule".

The term "nucleotide" refers to a molecule including a sugar, at least one phosphate group, and a nucleobase. Therefore, for ease of understanding, the "nucleotide" may be replaced by "base" in the embodiments of the present application. The nucleotides include deoxyribonucleotides, modified deoxyribonucleotides, ribonucleotides, modified ribonucleotides, peptide nucleotides, modified peptide nucleotides, modified sugar-phosphate backbone nucleotides, and mixtures thereof. Examples of the nucleotide include adenosine monophosphate (AMP), adenosine diphosphate (ADP), adenosine triphosphate (ATP), thymidine monophosphate (TMP), thymidine diphosphate (TDP), thymidine triphosphate (TTP), cytidine monophosphate (CMP), cytidine diphosphate (CDP), cytidine triphosphate (CTP), guanosine monophosphate (GMP), guanosine diphosphate (GDP), guanosine triphosphate (GTP), uridine monophosphate (UMP), uridine diphosphate (UDP), uridine triphosphate (UTP), deoxyadenosine monophosphate (dAMP), deoxyadenosine diphosphate (dADP), deoxyadenosine triphosphate (dATP), deoxythymidine monophosphate (dTMP), deoxythymidine diphosphate (dTDP), deoxythymidine triphosphate (dTTP), deoxycytidine diphosphate (dCDP), deoxycytidine triphosphate (dCTP), deoxyguanosine monophosphate (dGMP), deoxyguanosine diphosphate (dGDP), deoxyguanosine triphosphate (dGTP), deoxyuridine monophosphate (dUMP), deoxyuridine diphosphate (dUDP), and deoxyuridine triphosphate (dUTP).

As used herein, the term "nucleotide" also encompasses any nucleotide analogs that are those including a modified nucleobase, sugar, and/or phosphate moiety as compared to naturally occurring nucleotides. Exemplary modified nucleobases include inosine, xathanine, hypoxanthine, isocytosine, isoguanine, 2-aminopurine, 5-methylcytosine, 5-hydoxymethylcytosine, 2-aminoadenine, 6-methyladenine, 6-methylguanine, 2-propylguanine, 2-propyladenine, 2-thiouracil, 2-thiothymine, 2-thiocytosine, 15-halouracil, 15-halocytosine, 5-propynyluracil, 5-propynylcytosine, 6-azouracil, 6-azocytosine, 6-azothymine, 5-uracil, 4-thiouracil, 8-haloadenine or guanine, 8-aminoadenine or guanine, 8-thioladenine or guanine, 8-sulfanyladenine or guanine, 8-hydoxyadenine or guanine, 5-halouracil or cytosine, 7-methylguanine, 7-methyladenine, 8-azaguanine, 8-azaadenine, 7-deazaguanine, 7-deazaadenine, 3-deazaguanine, 3-deazaadenine, etc. As is known in the art, certain nucleotide analogs cannot be incorporated into polynucleotides, for example, nucleotide analogs such as adenosine 5'-phosphosulfate. The nucleotide may include any suitable number of phosphates, for example, three, four, five, six, or more than six phosphates.

The following disclosure provides many different embodiments or examples for implementing different structures of the present application. To simplify the disclosure of the present application, components and settings of specific examples are described below. Certainly, the examples are merely exemplary and are not intended to limit the present application. In addition, the present application may repeat reference numerals and/or reference letters in different examples. Such repetition is intended for simplicity and clarity rather than for indicating the relationship between various embodiments and/or settings discussed. In addition, the present application provides examples of various specific processes and materials, but those of ordinary skill in the art will recognize the application of other processes and/or the use of other materials.

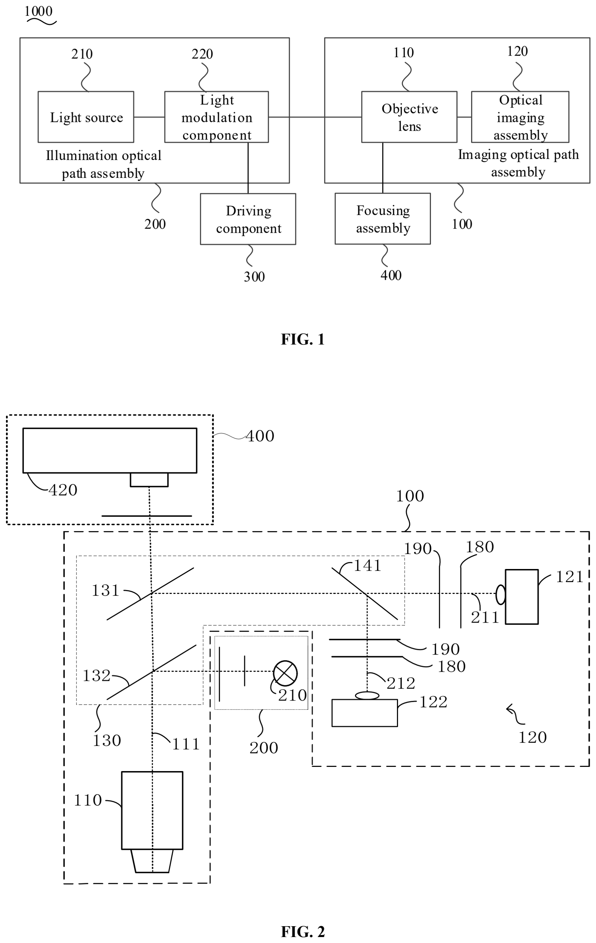

Referring to FIG. 1, the optical system 1000 according to the embodiments of the present application includes an imaging optical path assembly 100, an illumination optical path assembly 200, and a driving component 300. The imaging optical path assembly 100 is configured for acquiring an optical signal and generating an image. The illumination optical path assembly 200 is configured for providing an illumination light field and exciting an optical signal in a field of view. The driving component 300 is configured for driving a light modulation component 220 and/or a sample to move relative to an objective lens 110. Specifically, the illumination optical path assembly 200 provides the illumination light field, the driving component 300 drives the light modulation component 220 to modulate the illumination light field, and the imaging optical path assembly 100 receives an optical signal generated by the sample of interest through the illumination light field and generates an image.

It will be appreciated that, in the embodiments of the present application, the optical system 1000 may be configured for performing optical processing on a sample arranged on an optical path of the optical system 1000. The sample is a sample with an optical property. The optical property may be interpreted as that the sample has a luminescent property, that is, the sample may generate an optical signal; or may be interpreted as a property that the sample is capable of generating an optical signal in a specific condition when the sample is under a stress or undergoes a specific reaction. In some embodiments, the sample may be the sample itself, and in this case, the sample is a sample of interest having a certain solid shape; in some embodiments, the sample includes the sample of interest and a solid carrier for carrying the sample of interest; illustratively, the sample is a biochip or a sequencing chip, which, however, is not limiting.

The rationale of the optical system 1000 is as follows: The illumination light field provided by the illumination optical path assembly 200 reaches the surface of the sample through some elements of the imaging optical path assembly 100 and excites substances or structures on the sample to generate an optical signal such as a fluorescence signal, and the imaging optical path assembly 100 further captures the optical signal, and possibly further processes the captured optical signal. The further processing includes outputting data acquired by converting the optical signal into another form, for example, an optical image.

Referring again to FIG. 1, in some certain embodiments, the imaging optical path assembly 100 may include an objective lens 110 and an optical imaging assembly 120. The objective lens 110 is configured for projecting light emitted by the illumination optical path assembly 200 onto the sample and receiving the optical signal generated by the sample, and the optical imaging assembly 120 is configured for receiving the optical signal and forming an image.

Referring to FIG. 2, in some certain embodiments, the imaging optical path assembly 100 further includes a splitter mechanism 130 arranged between the objective lens 110 and the optical imaging assembly 120, where the splitter mechanism 130 is configured for splitting the optical signal into different light beams. The optical imaging assembly 120 includes a plurality of cameras, and each of the cameras is configured for receiving one of the light beams and generating an image.

As such, the plurality of cameras can receive different light beams and form images while acquiring different optical signals generated in the sample, thereby improving the efficiency of optical signal acquisition in the sample. For example, when the sample is a sequencing chip, the sequencing chip is a biochip carrying a nucleic acid template. Nucleotides (or nucleotide analogs, hereinafter referred to as "bases" for simplicity) containing fluorophores are bound to the nucleic acid template by a polymerization reaction. Fluorescence signals generated by the bases are divided by the splitter mechanism 130 into a preset number of different light beams with preset properties, and the preset number of different light beams with preset properties are separately acquired by different cameras. As such, different optical signals generated by various bases in the sample can be separately acquired by different cameras, resulting in higher information acquisition efficiency during the sequencing.

On this basis, properly setting the plurality of cameras at different spatial positions can make the imaging optical path assembly 100 compact in structure and reduce the volume of the imaging optical path assembly 100, featuring an improved space utilization rate.

Specifically, the objective lens 110 is configured for receiving the light from the sample of interest and transmitting the light to the splitter mechanism 130. The splitter mechanism 130 is configured for receiving the light from the objective lens 110 and splitting the light to form different light beams corresponding to different cameras. The cameras can convert the different light beams into two-dimensional images as raw data for subsequent data processing and analysis in the gene sequencing technology.

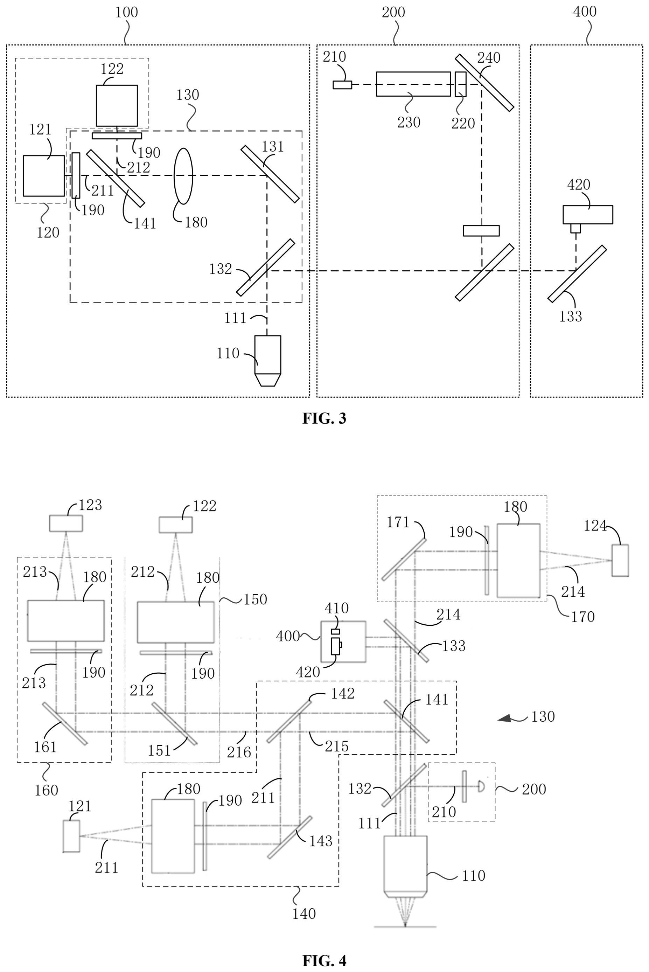

Referring again to FIGs. 2 and 3, in some certain embodiments, the splitter mechanism 130 is configured for splitting the optical signal generated by the sample into a first light beam 211 and a second light beam 212. The optical imaging assembly 120 includes a first camera 121 and a second camera 122 spaced apart from the first camera 121. The first camera 121 is configured for receiving the first light beam 211 and generating an image, and the second camera 122 is configured for receiving the second light beam 212 and generating an image.

As such, the first camera 121 and the second camera 122 can separately generate an image, so as to detect different lights emitted by the sample of interest. The two cameras can simultaneously generate images for at least two types of fluorescence signals, which can improve the imaging efficiency for various fluorescence signals.

For example, when the sample is a sequencing chip, the first camera 121 can generate images for fluorescence signals of bases A and/or G, and the second camera 122 can generate images for fluorescence signals of bases T and/or C.

Referring to FIGs. 2 and 3, in some certain embodiments, the splitter mechanism 130 includes a first dichroic mirror 141, and the first dichroic mirror 141 is configured for transmitting the optical signal to form the first light beam 211 and reflecting the optical signal to form the second light beam 212.

As such, the first dichroic mirror 141 can turn the light, such that the optical imaging assembly 120 and the objective lens 110 can be arranged at different positions, thus making the structure of the imaging optical path assembly 100 more compact. In addition, the first dichroic mirror 141 can separate optical signals of different wavebands, such that the first camera 121 and the second camera 122 can generate images on the basis of different optical signals.

It should be noted that the first light beam 211 and the second light beam 212 may have different wavebands. As shown in FIGs. 2 and 3, in some certain embodiments, the first dichroic mirror 141 is arranged aside from the optical axis 111 of the objective lens 110.

As such, the arrangement of the first dichroic mirror 141 on one side of the optical axis 111 of the objective lens 110 can prevent the arrangement of the imaging optical path assembly 100 on the optical axis of the objective lens 110, thereby reducing the dimension of the imaging optical path assembly 100 in a certain direction and making the structure of the imaging optical path assembly 100 more compact.

Specifically, in some embodiments, the first dichroic mirror 141 is arranged at an included angle of 45° relative to the optical axis 111 of the objective lens 110.

Referring to FIG. 3, in some certain embodiments, the splitter mechanism 130 further includes a mirror 131 arranged on the optical axis 111 of the objective lens 110, and the mirror 131 is arranged between the objective lens 110 and the first dichroic mirror 141 and configured for reflecting the optical signal to the first dichroic mirror 141.

As such, the mirror 131 can turn the light, such that the optical imaging assembly 120 and the objective lens 110 can be arranged at different positions, thus making the structure of the imaging optical path assembly 100 more compact. It should be noted that the mirror 131 may be a dichroic mirror.

Referring to FIG. 3, in some certain embodiments, the mirror 131 is arranged parallel or perpendicular to the first dichroic mirror 141. As such, the mirror 131 can change the direction of the second light beam 212, such that the optical imaging assembly 120 and the objective lens 110 can be arranged at different positions, thus making the structure of the imaging optical path assembly 100 more compact.

As shown in FIG. 3, when the mirror 131 is arranged parallel to the first dichroic mirror 141, the first light beam 211 is perpendicular to the optical axis 111 of the objective lens 110, and the second light beam 212 is in a direction identical to that of the light from the objective lens 110. As shown in FIG. 2, when the mirror 131 is arranged perpendicular to the first dichroic mirror 141, the first light beam 211 is perpendicular to the optical axis 111 of the objective lens 110, and the second light beam 212 is in a direction opposite to that of the light from the objective lens 110.

Referring to FIG. 3, in some certain embodiments, the imaging optical path assembly 100 includes an illumination dichroic mirror 132 arranged on the optical axis 111 of the objective lens 110. The illumination dichroic mirror 132 is arranged between the mirror 131 and the objective lens 110 and configured for reflecting the illumination light field to the objective lens 110 and transmitting the optical signal.

As such, the illumination dichroic mirror 132 reflects the light beam emitted by the illumination optical path assembly 200 to the objective lens 110, such that the light beam irradiates the sample of interest after passing through the objective lens 110 to excite the sample of interest to generate fluorescence with various wavelengths. The illumination dichroic mirror 132 is further configured for transmitting the light beam received by the objective lens 110 from the sample of interest to the optical imaging assembly 120, such that the optical imaging assembly 120 receives and acquires the corresponding light beam and forms a corresponding image. As shown in FIG. 2, the illumination dichroic mirror 132 is arranged parallel to the mirror 131, and as shown in FIG. 3, the illumination dichroic mirror 132 is arranged perpendicular to the mirror 131.

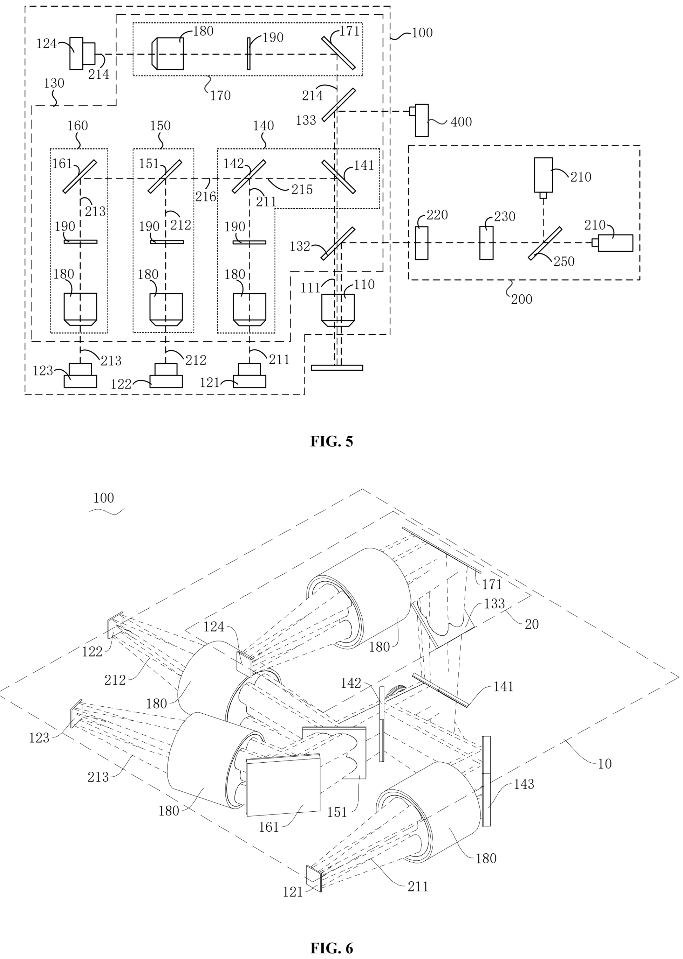

Referring to FIGs. 4 and 5, in some certain embodiments, the splitter mechanism 130 is configured for splitting the optical signal into a first light beam 211, a second light beam 212, a third light beam 213, and a fourth light beam 214. The optical imaging assembly 120 includes a first camera 121, a second camera 122, a third camera 123, and a fourth camera 124. The first camera 121 is configured for receiving the first light beam 211 and generating an image, the second camera 122 is configured for receiving the second light beam 212 and generating an image, the third camera 123 is configured for receiving the third light beam 213 and generating an image, and the fourth camera 124 is configured for receiving the fourth light beam 214 and generating an image.

As such, the use of the four cameras to receive different light beams and form images at different spatial positions not only makes the structure of the imaging optical path assembly 100 compact, reduces the volume of the imaging optical path assembly 100, and improves the space utilization rate, but also permits the simultaneous acquisition of a plurality of different optical signals in the sample, such as fluorescence signals corresponding to a plurality of bases in a sequencing chip, so as to improve the efficiency of information acquisition during the sequencing.

For example, the first camera 121 can generate an image on the basis of the fluorescence signal of base A, the second camera 122 can generate an image on the basis of the fluorescence signal of base T, the third camera 123 can generate an image on the basis of the fluorescence signal of base G, and the fourth camera 124 can generate an image on the basis of the fluorescence signal of base C.

Referring again to FIGs. 4 and 5, in some certain embodiments, the splitter mechanism 130 includes a first splitter mechanism 140, a second splitter mechanism 150, a third splitter mechanism 160, and a fourth splitter mechanism 170. The first splitter mechanism 140 splits the optical signal to form a first mixed light beam 215 and the fourth light beam 214. The first splitter mechanism 140 is further configured for splitting a portion of the first mixed light beam 215 to form the first light beam 211, converging the first light beam 211 to the first camera 121, and transmitting another portion of the first mixed light beam 215 as a second mixed light beam 216 to the second splitter mechanism 150. The second splitter mechanism 150 is configured for receiving the second mixed light beam 216, splitting the second mixed light beam 216 to form the second light beam 212 and the third light beam 213, converging the second light beam 212 to the second camera 122, and transmitting the third light beam 213 to the third splitter mechanism 160. The third splitter mechanism 160 is configured for converging the third light beam 213 to the third camera 123. The fourth splitter mechanism 170 receives the fourth light beam 214 formed by the first splitter mechanism 140 and converges the fourth light beam 214 to the fourth camera 124.

As such, the splitter mechanism 130 splits the light from the objective lens 110 to form a plurality of light beams and converges the light beams to corresponding cameras for imaging, so as to achieve the detection of different lights emitted by the sample of interest.

Referring to FIGs. 4 and 5, in some certain embodiments, the first splitter mechanism 140 includes a first dichroic mirror 141 and a second dichroic mirror 142. The first dichroic mirror 141 is arranged on the optical axis 111 of the objective lens 110, placed obliquely relative to the optical axis 111 of the objective lens 110, and configured for reflecting a light from the objective lens 110 to form the first mixed light beam 215 and transmitting the light from the objective lens 110 to form the fourth light beam 214. The second dichroic mirror 142 is spaced apart from the first dichroic mirror 141 and configured for reflecting a portion of the first mixed light beam 215 to form the first light beam 211 and directing the first light beam 211 to the first camera 121, and transmitting another portion of the first mixed light beam 215 to form the second mixed light beam 216.

As such, the first dichroic mirror 141 and the second dichroic mirror 142 can turn the light, such that the first camera 121, the fourth camera 124, and the objective lens 110 can be arranged at different positions, thus making the structure of the imaging optical path assembly 100 more compact.

Referring to FIG. 4, in some embodiments, the first dichroic mirror 141 is arranged on the optical axis 111 of the objective lens 110 at an included angle of 45° relative to the optical axis 111 of the objective lens 110. The first dichroic mirror 141 can split the light from the objective lens 110 to form the first mixed light beam 215 and the fourth light beam 214. The first mixed light beam 215 is perpendicular to the light from the objective lens 110, and the fourth light beam 214 is in a direction identical to that of the light from the objective lens 110. The first dichroic mirror 141 and the second dichroic mirror 142 are arranged at an included angle. The second dichroic mirror 142 can split the first mixed light beam 215 into the second mixed light beam 216 and the first light beam 211.

Referring to FIG. 5, in some certain embodiments, the included angle between the first dichroic mirror 141 and the second dichroic mirror 142 is 90° ± 15°.

Specifically, the included angle between the first dichroic mirror 141 and the second dichroic mirror 142 may be 75°, 80°, 85°, 90°, 95°, 100°, 105°, etc.

Referring to FIG. 4, in some certain embodiments, the first dichroic mirror 141 is arranged perpendicular to the second dichroic mirror 142.

As such, the second mixed light beam 216 is in a direction identical to that of the first mixed light beam 215, and the first light beam 211 is perpendicular to the first mixed light beam 215.

Referring again to FIG. 4, in some certain embodiments, the first splitter mechanism 140 further includes a first mirror 143. The first mirror 143 is spaced apart from the second dichroic mirror 142 and configured for reflecting a portion of the first light beam 211 from the second dichroic mirror 142 to the first camera 121. As such, the first mirror 143 can change the optical path of the first light beam 211, such that the first camera 121 can be arranged in a proper position to make the structure of the imaging optical path assembly 100 more compact.

Specifically, the first mirror 143 is arranged parallel to the second dichroic mirror 142, and the first light beam 211 is reflected by the first mirror 143 into a direction identical to that of the first mixed light beam 215.

Referring again to FIGs. 4 and 5, in some certain embodiments, the second splitter mechanism 150 includes a third dichroic mirror 151. The third dichroic mirror 151 is spaced apart from the second dichroic mirror 142, arranged on the side of the second dichroic mirror 142 distal to the first dichroic mirror 141, and configured for reflecting a portion of the second mixed light beam 216 to form the second light beam 212 and directing the second light beam 212 to the second camera 122, and transmitting another portion of the second mixed light beam 216 to form the third light beam 213.

As such, the third dichroic mirror 151 can split the second mixed light beam 216 into a second light beam 212 and a third light beam 213, such that the second camera 122 and the third camera 123 can be arranged at different positions, thus making the structure of the imaging optical path assembly 100 more compact. Referring to FIG. 5, in some certain embodiments, the second dichroic mirror 142 is substantially parallel to the third dichroic mirror 151.

As such, the second light beam 212 is perpendicular to the second mixed light beam 216, such that the second light beam 212 and the first light beam 211 are on the same side of the second mixed light beam 216. Referring to FIG. 4, in some certain embodiments, the included angle between the third dichroic mirror 151 and the second dichroic mirror 142 is 90° ± 15°.

Specifically, the included angle between the third dichroic mirror 151 and the second dichroic mirror 142 may be 75°, 80°, 85°, 90°, 95°, 100°, 105°, etc.

Referring again to FIG. 4, in some certain embodiments, the third dichroic mirror 151 is arranged perpendicular to the second dichroic mirror 142.

As such, the second light beam 212 is perpendicular to the second mixed light beam 216, such that the second light beam 212 and the first light beam 211 are separately located on two sides of the second mixed light beam 216.

Referring again to FIGs. 4 and 5, in some certain embodiments, the third splitter mechanism 160 includes a second mirror 161. The second mirror 161 is spaced apart from the third dichroic mirror 151, arranged on the side of the third dichroic mirror 151 distal to the second dichroic mirror 142, and configured for reflecting the third light beam 213 to the third camera 123.

As such, the second mirror 161 can change the optical path of the third light beam 213, such that the third light beam 213 is perpendicular to the second mixed light beam 216 through the second mirror 161.

In some certain embodiments, the second mirror 161 is arranged parallel or perpendicular to the third dichroic mirror 151.

As such, the third light beam 213 is parallel to the second light beam 212 and perpendicular to the second mixed light beam 216.

As shown in FIG. 4, the second mirror 161 is arranged parallel to the third dichroic mirror 151, the second light beam 212 is in a direction identical to that of the third light beam 213, and the second camera 122 and the third camera 123 are located on the same side of the third plane 30. In other embodiments, the second mirror 161 is arranged perpendicular to the third dichroic mirror 151, and the second light beam 212 is in a direction opposite to that of the third light beam 213.

Referring again to FIGs. 4 and 5, in some certain embodiments, the fourth splitter mechanism 170 includes a third mirror 171. The third mirror 171 is arranged on the optical axis 111 of the objective lens 110 and obliquely relative to the optical axis 111 of the objective lens 110, located on the side of the first dichroic mirror 141 distal to the objective lens 110, and configured for reflecting the fourth light beam 214 to the fourth camera 124.

As such, the third mirror 171 is configured for changing the optical path of the fourth light beam 214, such that the fourth light beam 214 is perpendicular to the optical axis 111 of the objective lens 110 through the third mirror 171. Specifically, the third mirror 171 is located on the optical axis 111 of the objective lens 110 and arranged parallel or perpendicular to the first dichroic mirror 141.

Referring to FIGs. 4 and 6, FIG. 6 is a schematic perspective view of the imaging optical path assembly 100 according to the embodiment in FIG. 4. In some certain embodiments, the first camera 121, the second camera 122, and the third camera 123 are located on a first plane 10. The first plane 10 intersects the optical axis 111 of the objective lens 110. The fourth camera 124 is located on one side of the first plane 10.

The first plane 10 may be a plane formed by the three center points of the first camera 121, the second camera 122, and the third camera 123, and the optical axis 111 of the objective lens 110 intersects the first plane 10. Specifically, as relative to the first plane 10, the objective lens 110 may be located below the first plane 10, and the fourth camera 124 may be located above the first plane 10.

In some certain embodiments, the first plane 10 is perpendicular to the optical axis 111 of the objective lens 110.

As such, the first light beam 211, the second light beam 212, and the third light beam 213 are all perpendicular to the optical axis 111 of the objective lens 110, so as to reduce the turns of the light and make the structure of the imaging optical path assembly 100 compact.

Referring again to FIG. 6, in some certain embodiments, the fourth camera 124 is located on a second plane 20 parallel to the first plane 10.

As such, the fourth light beam 214 is perpendicular to the optical axis 111 of the objective lens 110, so as to reduce the turns of the light and make the structure of the imaging optical path assembly 100 compact.

In some certain embodiments, the normal direction of the imaging surface of the first camera 121 and the normal direction of the imaging surface of the fourth camera 124 are identical or opposite.

As such, the first light beam 211 is parallel to the fourth light beam 214, and the two are both perpendicular to the optical axis 111 of the objective lens 110, so as to reduce the turns of the light and make the structure of the imaging optical path assembly 100 compact.

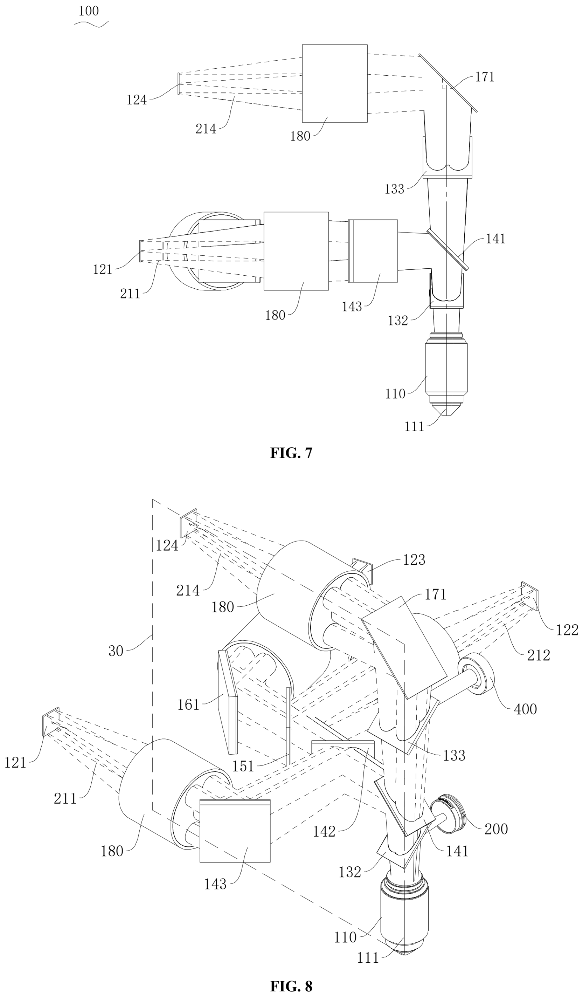

As shown in FIG. 4, the normal direction of the imaging surface of the first camera 121 and the normal direction of the imaging surface of the fourth camera 124 are opposite, and the first camera 121 and the fourth camera 124 are located on two sides of the optical axis 111 of the objective lens 110. As shown in FIG. 7, in the illustrated embodiment, the normal direction of the imaging surface of the first camera 121 and the normal direction of the imaging surface of the fourth camera 124 are identical, and the first camera 121 and the fourth camera 124 are located on the same side of the optical axis 111 of the objective lens 110. Referring to FIG. 4, in some certain embodiments, the normal direction of the imaging surface of the second camera 122 and the normal direction of the imaging surface of the third camera 123 are identical.

As such, the second light beam 212 and the third light beam 213 are located in the first plane 10 and arranged parallel to each other and both perpendicular to the optical axis 111 of the objective lens 110, so as to reduce the turns of the light and make the structure of the imaging optical path assembly 100 compact.

As shown in FIG. 4, the normal direction of the imaging surface of the second camera 122 and the normal direction of the imaging surface of the third camera 123 are identical, and the second camera 122 and the third camera 123 are located on the same side of the optical axis 111 of the objective lens 110. In other embodiments, the normal direction of the imaging surface of the second camera 122 and the normal direction of the imaging surface of the third camera 123 may be opposite, and the second camera 122 and the third camera 123 are located on two sides of the optical axis 111 of the objective lens 110.

In some certain embodiments, the distance from the center of the fourth camera 124 to the optical axis 111 of the objective lens 110 is less than that from the center of the first camera 121 to the optical axis 111 of the objective lens 110. As such, by reducing the distance from the center of the first camera 121 to the optical axis 111 of the objective lens 110, the volume of the apparatus can be reduced, thus making the structure of the imaging optical path assembly 100 compact.

In some certain embodiments, the distance from the center of the second camera 122 to the optical axis 111 of the objective lens 110 is less than that from the center of the third camera 123 to the optical axis 111 of the objective lens 110. As such, by reducing the distance from the center of the third camera 123 to the optical axis 111 of the objective lens 110, the volume of the apparatus can be reduced, thus making the structure of the imaging optical path assembly 100 compact.

Referring to FIG. 8, in some certain embodiments, the first camera 121 and the second camera 122 are separately located on two sides of the third plane 30, and the third plane 30 passes through the optical axis 111 of the objective lens 110 and the center of the fourth camera 124.

The third plane 30 is a plane formed by the optical axis 111 of the objective lens 110 and the center of the fourth camera 124. The first camera 121 and the second camera 122 are separately located on two sides of the third plane 30, and the second camera 122 and the third camera 123 are located on the same side of the third plane 30.

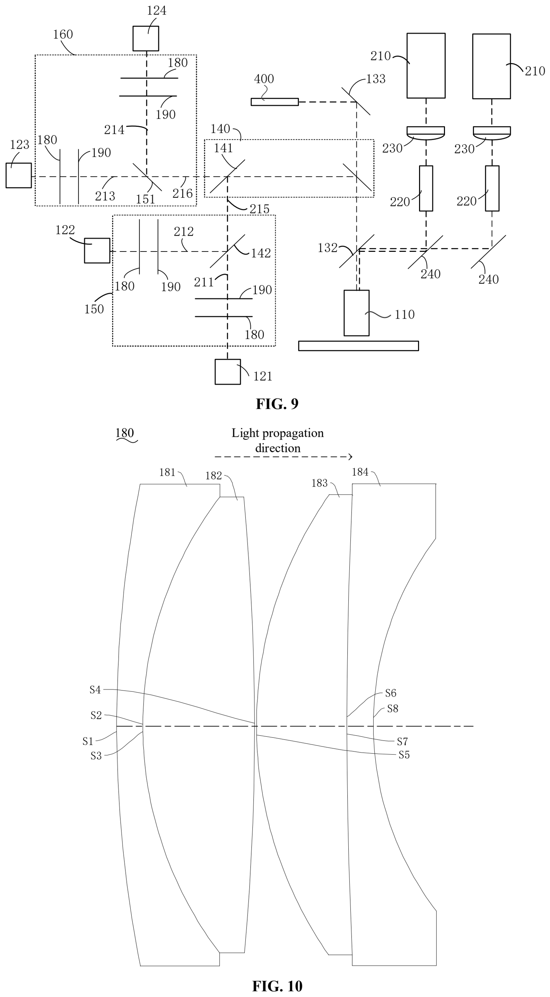

Referring to FIG. 9, in some certain embodiments, the splitter mechanism 130 includes a first splitter mechanism 140, a second splitter mechanism 150, and a third splitter mechanism 160. The first splitter mechanism 140 splits the optical signal to form a first mixed light beam 215 and a second mixed light beam 216. The second splitter mechanism 150 is configured for splitting the first mixed light beam 215 to form the first light beam 211 and the second light beam 212, and converging the first light beam 211 to the first camera 121 and the second light beam 212 to the second camera 122. The third splitter mechanism 160 is configured for splitting the second mixed light beam 216 to form the third light beam 213 and the fourth light beam 214, and converging the third light beam 213 to the third camera 123 and the fourth light beam 214 to the fourth camera 124.

As such, the splitter mechanism 130 splits the light from the objective lens 110 to form a plurality of light beams and converges the light beams to corresponding cameras for imaging, so as to achieve the detection of different lights emitted by the sample of interest.

Referring again to FIG. 9, in some certain embodiments, a plurality of light sources 210 are provided. Different light sources 210 emit lights of different wavebands, and the lights of different wavebands excite optical signals of different wavebands. The first camera 121, the second camera 122, the third camera 123, and the fourth camera 124 are configured for generating an image according to the optical signals of different wavebands.

As such, the plurality of light sources 210 can excite fluorophores on different bases, thereby achieving the sequencing of various bases. Specifically, the number of the light sources 210 may be 2, 3, 4, or the like. The plurality of light sources 210 are combined by a beam combiner component, and the beam combiner component may be a dichroic mirror. For example, when the sample is a sequencing chip, the first camera 121 can generate an image on the basis of the fluorescence signal of base A, the second camera 122 can generate an image on the basis of the fluorescence signal of base T, the third camera 123 can generate an image on the basis of the fluorescence signal of base G, and the fourth camera 124 can generate an image on the basis of the fluorescence signal of base C. When the number of the light sources 210 is two, one of the light sources 210 can excite the fluorescence signal of base A and the fluorescence signal of base T, and the other light source 210 can excite the fluorescence signal of base C and the fluorescence signal of base G.

Referring to FIG. 9, in some certain embodiments, the first splitter mechanism 140 includes a first dichroic mirror 141, the second splitter mechanism includes a second dichroic mirror 142, and the third splitter mechanism 160 includes a third dichroic mirror 151. The first dichroic mirror 141 splits the optical signal to form a first mixed light beam 215 and a second mixed light beam 216. The second dichroic mirror 142 is configured for splitting the first mixed light beam 215 to form the first light beam 211 and the second light beam 212, and converging the first light beam 211 to the first camera 121 and the second light beam 212 to the second camera 122. The third dichroic mirror 151 is configured for splitting the second mixed light beam 216 to form the third light beam 213 and the fourth light beam 214, and converging the third light beam 213 to the third camera 123 and the fourth light beam 214 to the fourth camera 124.

As such, the first dichroic mirror 141, the second dichroic mirror 142, and the third dichroic mirror 151 can turn the light, such that the first camera 121, the second camera 122, the third camera 123, the fourth camera 124, and the objective lens 110 can be arranged at different positions, thus making the structure of the imaging optical path assembly 100 more compact.

As shown in FIG. 9, in some certain embodiments, the first dichroic mirror 141 is parallel or perpendicular to the second dichroic mirror 142.

As such, the direction of light beams can be changed, such that the optical imaging assembly 120 and the objective lens 110 can be arranged at different positions, thus making the structure of the imaging optical path assembly 100 more compact.

Specifically, the second dichroic mirror 142 is configured for transmitting a portion of the first mixed light beam 215 to form the first light beam 211 and reflecting another portion of the first mixed light beam 215 to form the second light beam 212. As shown in FIG. 9, the second dichroic mirror 142 is arranged parallel to the first dichroic mirror 141, the first light beam 211 is perpendicular to the first mixed light beam 215, and the second light beam 212 is in a direction identical to that of the first mixed light beam 215. In some embodiments, the second dichroic mirror 142 is arranged perpendicular to the first dichroic mirror 141, the first light beam 211 is perpendicular to the first mixed light beam 215, and the second light beam 212 is in a direction opposite to that of the first mixed light beam 215.

Referring to FIG. 9, in some certain embodiments, the first dichroic mirror 141 is arranged aside from the optical axis 111 of the objective lens 110.

As such, the arrangement of the first dichroic mirror 141 on one side of the optical axis 111 of the objective lens 110 can prevent the arrangement of the imaging optical path assembly 100 on the optical axis of the objective lens 110, thereby reducing the dimension of the imaging optical path assembly 100 in a certain direction and making the structure of the imaging optical path assembly 100 more compact.

Specifically, in some embodiments, the first dichroic mirror 141 is arranged at an included angle of 45° relative to the optical axis 111 of the objective lens 110.

In some certain embodiments, the imaging optical path assembly 100 further includes a tube lens 180 arranged on the object-side optical path of the camera and configured for generating an image on the camera. Referring again to FIGs. 4, 5, and 9, the imaging optical path assembly 100 according to embodiments of the present application includes the tube lens 180 cooperating with the camera. A proper design of the surface form and the refractive power of each lens in the tube lens 180 may help achieve a high-quality imaging effect of the camera. Specifically, the tube lens 180 is configured for cooperating with the objective lens 110 to converge the light beams to the camera for imaging. Tube lenses 180 with different focal lengths can be designed according to the imaging requirements.

Referring again to FIGs. 3 to 5 and 9, in some certain embodiments, the tube lens 180 is located between the camera and the splitter mechanism 130 or in the internal optical path of the splitter mechanism 130. As such, the tube lens 180 can converge the light beam after the splitting, and may also converge the light beam inside the splitter mechanism 130, so as to assist the imaging of the light beams on the cameras.

As shown in FIG. 5, when the tube lens 180 is located between the camera and the splitter mechanism 130, the light beams are converged to the camera for imaging after the splitting. As shown in FIG. 3, when the tube lens 180 is located on the internal optical path of the splitter mechanism 130, the light beams can be converged and then split.

Referring to FIG. 10, in some certain embodiments, the tube lens 180 sequentially includes a first lens 181, a second lens 182, a third lens 183, and a fourth lens 184. The first lens 181 has a negative refractive power, the second lens 182 has a positive refractive power, the third lens 183 has a positive refractive power, and the fourth lens 184 has a negative refractive power. The object-side surfaces and the image-side surfaces of the first lens 181, the second lens 182, the third lens 183, and the fourth lens 184 are all spherical surfaces. The object-side surface of one lens and the image-side surface of the other lens in every pair of adjacent lenses are arranged opposite.

As such, the tube lens 180 is in a four-lens structure, and the object-side surfaces and the image-side surfaces of the four lenses are all spherical surfaces, such that the tube lens 180 can greatly reduce the aberration in a visible wavelength range of 553 nm to 712 nm. The combination of the tube lens 180 with an infinite conjugate lens can sharply converge the base fluorescence spots of the object surface on the image surface of the camera.

In addition, the tube lens 180 has a reduced number of lenses and a reduced volume. The object-side surfaces and the image-side surfaces of the four lenses are all spherical surfaces. Compared with aspherical lenses, the lenses according to embodiments of the present application feature ease to manufacture, thereby making the manufacture of the tube lens 180 easier. The combination of the tube lens 180 with an infinite conjugate objective lens 110 can sharply converge base fluorescence spots of the object surface on the image surface of the camera.

Specifically, the first lens 181, the second lens 182, the third lens 183, and the fourth lens 184 are arranged in sequence along the light propagation direction (as indicated by the dashed arrow in FIG. 10).

The aforementioned "positive refractive power" indicates that the optical lens has a positive focal length and a light-converging effect. The "negative refractive power" indicates that the optical lens has a negative focal length and a light-diverging effect.

As relative to the lens, the side where the object is located is the object side, and the surface of the lens proximal to the object side is referred to as the object-side surface; the side where the image of the object is located is the image side, and the surface of the lens proximal to the image side is referred to as the image-side surface.

In some certain embodiments, the tube lens 180 satisfies the following relational formula (1):

$$\frac{\Delta l'_{FC}}{\beta^2} \leq \frac{DOF}{6} \quad ......(1)$$

where $\Delta l'_{FC}$ denotes the axial chromatic aberration of the tube lens 180, $\beta$ denotes the magnification factor of the tube lens 180, and DOF denotes the depth of field of the tube lens 180.

The above relational formula (1) is a tolerance portion allocated by the depth of field of the tube lens 180 to the axial chromatic aberration, which, in this condition, is desirable for a good definition of lights of various wavelengths at the image surface and the acquisition of optical signals of various wavebands in the same field of view (FOV).

Specifically, a light beam parallel to the optical axis 111 may be converged at different positions after passing through the lens. Such an imaging difference is referred to as axial chromatic aberration.

The depth of field refers to the object distance range that is measured along the axis of an imager in front of a lens or other imagers and can provide a clear image.

Referring again to FIG. 10, in some certain embodiments, the object-side surface S1 of the first lens 181 is a first convex surface at the optical axis 111, and the image-side surface S2 of the first lens 181 is a first concave surface at the optical axis 111. The object-side surface S1 of the first lens 181 has a curvature radius of 100 mm to 200 mm, and the image-side surface S2 of the first lens 181 has a curvature radius of 20 mm to 40 mm at the optical axis 111.

When the first lens 181 meets the curvature radius requirements, the first lens 181 can be prevented from an excessively flat or excessively bent profile, thereby reducing the design difficulty and the assembly sensitivity of the tube lens 180. In addition, it is desirable for reducing the angle at which the main light of the peripheral angle of view is incident to the imaging surface, inhibiting the astigmatism, and ensuring a sharp and uniform imaging effect from the center to the edge of the imaging surface.

The positivity or negativity of the curvature radius indicates that the optical surface is convex towards the object side or convex towards the image side. When an optical surface (including the object-side surface and the image-side surface) is convex towards the object side, the curvature radius of the optical surface is a positive value; when an optical surface (including the object-side surface and the image-side surface) is convex towards the image side, the optical surface is concave towards the object side, and the curvature radius of the optical surface is a negative value.

That is, the curvature radius of the object-side surface S1 of the first lens 181 may be any value in interval [100 mm, 200 mm]; for example, the value may be 100, 120, 140, 160, 180, 200, etc., in mm. The curvature radius of the image-side surface S2 of the first lens 181 may be any value in interval [20 mm, 40 mm]; for example, the value may be 20, 24, 28, 32, 36, 40, etc., in mm.

The configuration of the object-side surface S1 of the first lens 181 as a convex surface near the optical axis 111 can improve the light-converging capacity of the object-side surface and reduce the overall thickness of the tube lens 180. The configuration of the image-side surface S2 of the first lens 181 as a concave surface near the optical axis 111 can reduce the astigmatism of the tube lens 180, such that the tube lens 180 can greatly reduce the aberration in a visible wavelength range of 553 nm to 712 nm. The combination of the tube lens 180 with an infinite conjugate lens can sharply converge the base fluorescence spots of the object surface on the image surface of the camera.

Referring again to FIG. 10, in some certain embodiments, the object-side surface S3 of the second lens 182 is a second convex surface at the optical axis 111, and the image-side surface S4 of the second lens 182 is a third convex surface at the optical axis 111. The object-side surface S3 of the second lens 182 has a curvature radius of 20 mm to 40 mm at the optical axis 111, and the image-side surface S4 of the second lens 182 has a curvature radius of -250 mm to -200 mm at the optical axis 111.

When the second lens 182 meets the curvature radius requirements, the second lens 182 can be prevented from an excessively flat or excessively bent profile, thereby reducing the design difficulty and the assembly sensitivity of the tube lens 180. In addition, it is desirable for reducing the angle at which the main light of the peripheral angle of view is incident to the imaging surface, inhibiting the astigmatism, and ensuring a sharp and uniform imaging effect from the center to the edge of the imaging surface.

That is, the curvature radius of the object-side surface S3 of the second lens 182 may be any value in interval [20 mm, 40 mm]; for example, the value may be 20, 24, 28, 32, 36, 40, etc., in mm. The curvature radius of the image-side surface S4 of the second lens 182 may be any value in interval [-250 mm, -200 mm]; for example, the value may be -250, -240, -230, -220, -210, -200, etc., in mm.

Referring again to FIG. 10, in some certain embodiments, the object-side surface S5 of the third lens 183 is a fourth convex surface at the optical axis 111, and the image-side surface S6 of the third lens 183 is a second concave surface at the optical axis 111. The object-side surface S5 of the third lens 183 has a curvature radius of 20 mm to 40 mm at the optical axis 111, and the image-side surface S6 of the third lens 183 has a curvature radius of 400 mm to 500 mm at the optical axis 111.

When the third lens 183 meets the curvature radius requirements, the third lens 183 can be prevented from an excessively flat or excessively bent profile, thereby reducing the design difficulty and the assembly sensitivity of the tube lens 180. In addition, it is desirable for reducing the angle at which the main light of the peripheral angle of view is incident to the imaging surface, inhibiting the astigmatism, and ensuring a sharp and uniform imaging effect from the center to the edge of the imaging surface.

That is, the curvature radius of the object-side surface S5 of the third lens 183 may be any value in interval [20 mm, 40 mm]; for example, the value may be 20, 24, 28, 32, 36, 40, etc., in mm. The curvature radius of the image-side surface S6 of the third lens 183 may be any value in interval [400 mm, 500 mm]; for example, the value may be 400, 420, 440, 460, 480, 500, etc., in mm.

Referring again to FIG. 10, in some certain embodiments, the object-side surface S7 of the fourth lens 184 is a fifth convex surface at the optical axis 111, and the image-side surface S8 of the fourth lens 184 is a third concave surface at the optical axis 111. The object-side surface S7 of the fourth lens 184 has a curvature radius of 400 mm to 500 mm at the optical axis 111, and the image-side surface S8 of the fourth lens 184 has a curvature radius of 20 mm to 40 mm at the optical axis 111.

When the fourth lens 184 meets the curvature radius requirements, the fourth lens 184 can be prevented from an excessively flat or excessively bent profile, thereby reducing the design difficulty and the assembly sensitivity of the tube lens 180. In addition, it is desirable for reducing the angle at which the main light of the peripheral angle of view is incident to the imaging surface, inhibiting the astigmatism, and ensuring a sharp and uniform imaging effect from the center to the edge of the imaging surface.

That is, the curvature radius of the object-side surface S7 of the fourth lens 184 may be any value in interval [400 mm, 500 mm]; for example, the value may be 400, 420, 440, 460, 480, 500, etc., in mm. The curvature radius of the image-side surface S8 of the fourth lens 184 may be any value in interval [20 mm, 40 mm]; for example, the value may be 20, 24, 28, 32, 36, 40, etc., in mm.

In some certain embodiments, the first lens 181 has a thickness of 2.0 mm to 3.0 mm at the optical axis 111, the second lens 182 has a thickness of 9 mm to 12 mm at the optical axis 111, the third lens 183 has a thickness of 7 mm to 10 mm at the optical axis 111, and the fourth lens 184 has a thickness of 2.0 mm to 3.0 mm at the optical axis 111.

A proper configuration of the thicknesses of different lenses can keep an optimal balance between the miniaturization of the tube lens 180 and the manufacturability of the lens, thereby avoiding compromised strength of the tube lens 180 due to excessively thin lenses and thus affected yield. In addition, when the thicknesses of the lenses at the optical axis 111 are in the above ranges, the tube lens 180 can greatly reduce the aberration in a visible wavelength range of 553 nm to 712 nm. The combination of the tube lens 180 with an infinite conjugate lens can sharply converge the base fluorescence spots of the object surface on the image surface of the camera. That is, the thickness of the first lens 181 at the optical axis 111 may be any value in interval [2.0 mm, 3.0 mm]; for example, the value may be 2.0, 2.2, 2.4, 2.6, 2.8, 3.0, etc., in mm. The thickness of the second lens 182 at the optical axis 111 may be any value in interval [9.0 mm, 12.0 mm]; for example, the value may be 9.0, 9.5, 10.0, 10.5, 11.0, 11.5, 12.0, etc., in mm.

The thickness of the third lens 183 at the optical axis 111 may be any value in interval [7.0 mm, 10.0 mm]; for example, the value may be 7.0, 7.5, 8.0, 8.5, 9.0, 9.5, 10.0, etc., in mm.

The thickness of the fourth lens 184 at the optical axis 111 may be any value in interval [2.0 mm, 3.0 mm]; for example, the value may be 2.0, 2.2, 2.4, 2.6, 2.8, 3.0, etc., in mm.

In some embodiments, one or more additional lenses may be arranged between any two adjacent lenses. For example, a fifth lens (not shown in the drawings) is arranged between the second lens 182 and the third lens 183.

In some certain embodiments, the first lens 181 and the second lens 182 are cemented to form a lens set, and the third lens 183 and the fourth lens 184 are cemented to form a lens set.

Through the cemented lenses, the optical axes 111 of the first lens 181 and the second lens 182 are aligned, and the optical axes 111 of the third lens 183 and the fourth lens 184 are aligned, thus eliminating the chromatic aberration between the first lens 181 and the second lens 182 and the chromatic aberration between the third lens 183 and the fourth lens 184, and allowing the tube lens 180 to greatly reduce the aberration in a visible wavelength range of 553 nm to 712 nm. The combination of the tube lens 180 with an infinite conjugate lens can sharply converge the base fluorescence spots of the object surface on the image surface of the camera. The cemented lenses are also beneficial to the assembly and calibration of the tube lens 180.

In the manufacturing process of optical lenses, two lenses are usually cemented together by resin cementation or optical cementation, and the cement is cured to fix the lenses, so as to give cemented lens sets.

Specifically, the first lens 181, the second lens 182, the third lens 183, and the fourth lens 184 may be made of different materials. In addition, the first lens 181 may be made of flint glass, the second lens 182 may be made of crown glass, the third lens 183 may be made of flint glass, and the fourth lens 184 may be made of flint glass.

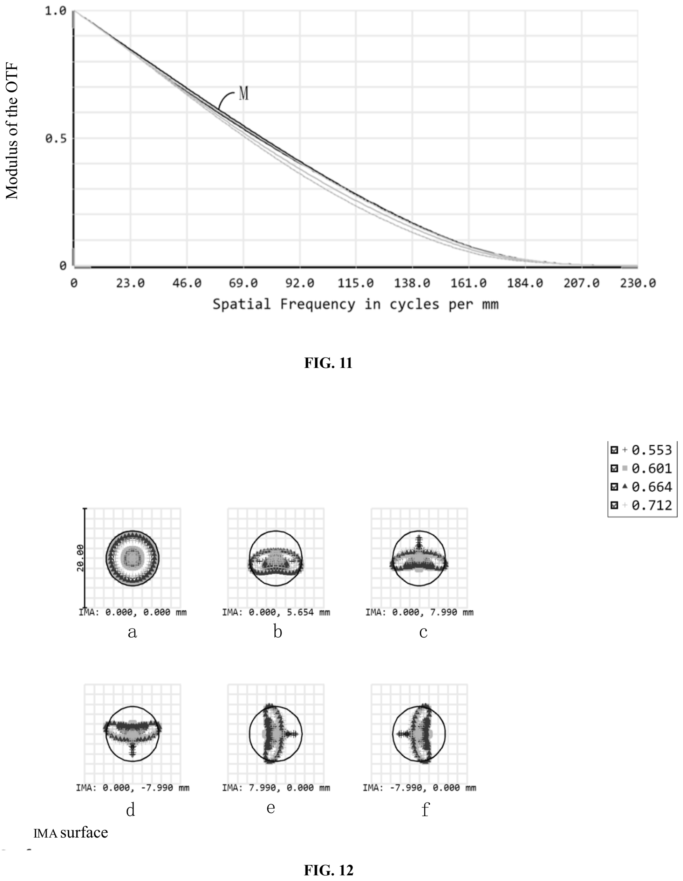

FIG. 11 shows MTF curves of a tube lens 180 according to the embodiments of the present application. The MTF curves can be used to evaluate the resolution indicators of the tube lens 180. In the figure, the abscissa denotes the spatial frequency, and the ordinate denotes the value of MTF. The maximal value is 1, and a greater value indicates a higher resolution. In different conditions, for example, at different temperatures, expansion coefficients of lenses made of different glass materials are different, which may lead to the potential of deformation. The curves shown in FIG. 11 include an MTF curve M of the diffraction limit and MTF curves of 6 other fields of view. It can be seen from the figure that the MTF curves of the other different fields of view are all close to the diffraction limit at different spatial frequencies. It can be seen that, in the tube lens 180 according to the embodiments of the present application, the fields of view are all close to the diffraction limit, suggesting that the optimal resolution is achieved.

FIG. 12 shows a spot diagram in 6 different fields of view of the tube lens 180 according to the embodiments of the present application. The spot diagram is a spot pattern observed at the image side of the spots in the field of view at the object side. The spot diagram has a diffraction-limited circle with an Airy radius of 5.514 $\mu$m, and the root mean square (RMS) radii of the spots in the fields of view coded a-f are 2.547 $\mu$m, 2.192 $\mu$m, 2.186 $\mu$m, 2.186 $\mu$m, 2.186 $\mu$m, and 2.186 $\mu$m, respectively. It can be seen that, in the tube lens 180 according to the embodiments of the present application, the spot patterns in the 6 fields of view are all within or close to the diffraction-limited circle. The spot size of the tube lens 180 reaches the diffraction limit in the fields of view, and a better image quality effect is achieved, suggesting good aberration of the optical system 1000 including the tube lens 180.

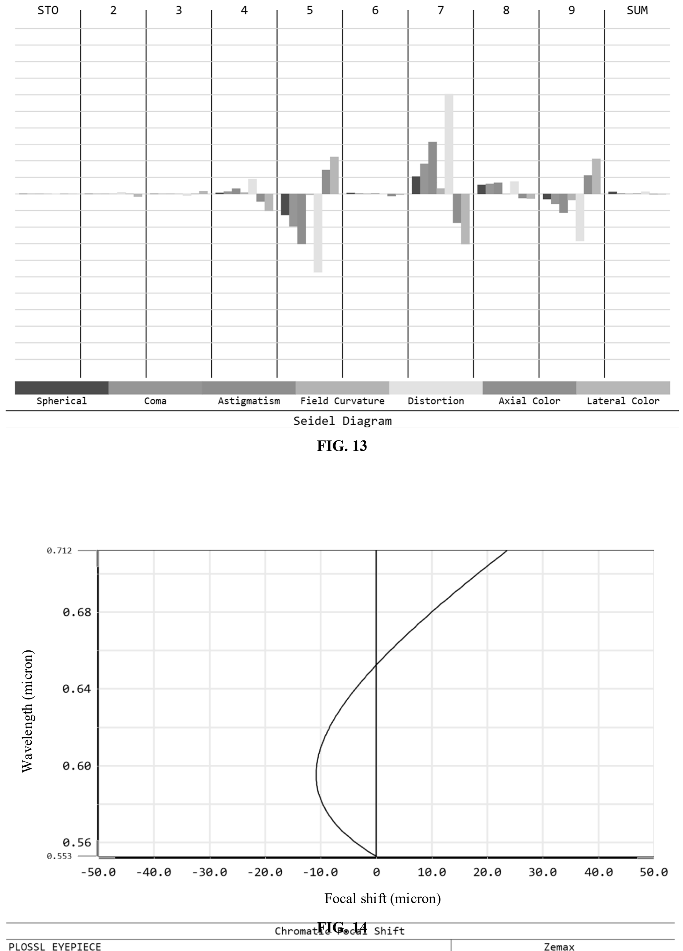

FIG. 13 shows a Seidel diagram of one implementation of the tube lens 180 according to the embodiments of the present application. The Seidel diagram can reflect the aberration introduced by the lenses in the optical system 1000, where STO denotes the system aberration introduced by the aperture stop, 2 and 3 denote the system aberration introduced by two surfaces of the filter arranged before the tube lens 180, 4 to 9 denote the system aberration introduced by the six surfaces (S2/S3 are cemented to form one surface, and S6/S7 are cemented to form one surface) of the tube lens 180, the sum denotes the synthesized system aberration after balancing the introduced aberrations of the lenses, the vertical axis denotes the magnitude of aberration, and the magnitude of aberration between adjacent (horizontal) grid lines is 0.01 mm. From the bars in the sum in FIG. 13, it can be seen that the tube lens 180 exhibits a uniform aberration distribution and a small synthesized aberration.

FIG. 14 is a diagram illustrating the focal shift curve of the tube lens 180 according to the embodiments of the present application. The focal shift diagram is a positional diagram of the focuses of light beams of different wavelengths. It can be seen from the figure that the maximum focal shift range, or axial chromatic aberration, from 553 nm to 712 nm is 34.3906 $\mu$m.

In some certain embodiments, the object-side field of view of the combination of the tube lens 180 with the objective lens 110 is greater than 1.0 mm. In one embodiment, the objective lens 110 is an infinite conjugate objective lens 110.

The above object-side field of view is beneficial to detecting a greater area on a sample, thereby allowing the system to provide more comprehensive and detailed measurement results and more information. As such, the tube lens 180 may fit a greater range of optical devices. It will be appreciated that the object-side field of view is a field of view at the side of a sample of interest after the tube lens 180 is combined with the objective lens 110. For example, the object-side field of view may be the diameter of a circular region that is actually and effectively visible on the sample of interest under the objective lens 110.

In some certain embodiments, the tube lens 180 satisfies the following relational formula (2):

$$120 \text{ mm} < f < 140 \text{ mm} \ldots\ldots(2)$$

where f denotes the effective focal length of the tube lens 180.

When the above relational formula (2) is satisfied, the tube lens 180 can effectively control the aberration, thereby improving imaging quality. Specifically, the effective focal length of the tube lens 180 matches the focal length of the objective lens 110 and the size of the camera pixel and satisfies certain magnification requirements, and the minimum spacing between the fluorescence spots on the object surface and the size of the camera pixel satisfy the Nyquist-Shannon sampling theorem, that is, the minimum spacing between the fluorescence spots $\times$ the magnification factor is greater than or equal to 2 times the size of the pixel. In the condition that the magnification of the imaging optical path assembly 100 remains unchanged, the effective focal length of the objective lens 110 may be directly proportional to the effective focal length of the tube lens 180. In addition, the magnification of the imaging optical path assembly 100 is equal to the ratio of the effective focal length of the tube lens 180 to the effective focal length of the objective lens 110. Illustratively, the focal length of the objective lens 110 may be 10 mm, such that the imaging optical path assembly 100 meets the design requirements of the illumination range and illumination uniformity in certain applications.

In some certain embodiments, the tube lens 180 has a back focal distance greater than or equal to 70 mm.

When the back focal distance meets the above requirement, the mechanical length of the tube lens 180 is reduced, which is beneficial to the miniaturization design of the tube lens 180. Specifically, the back focal distance is the distance between the surface center of the optical surface of the tube lens 180 closest to the optical imaging assembly 120 and the imaging surface of the optical imaging assembly 120.

Referring to FIG. 9, in some embodiments, the imaging optical path assembly 100 includes a filter 190. The filter 190 is arranged on the side of the tube lens 180 distal to the camera. The filter 190 can filter lights, allow the fluorescence wavebands to pass through, and cut off the undesired excitation waveband, thereby improving the imaging quality of the camera.

Referring to FIGs. 3 and 5, in some certain embodiments, the illumination optical path assembly 200 includes a light source 210 and a light modulation component 220. The light modulation component 220 is configured for transmitting and modulating the illumination light generated by the light source 210 to form an illumination light field.

As such, the light modulation component 220 can adjust the illumination light field, such that a super-resolution image is reconstructed more easily, thereby helping improve the reconstruction efficiency of the super-resolution image.

Specifically, the light source 210 is configured for emitting an illumination light. The illumination light irradiates a sample of interest and excites the sample of interest to generate optical signals. The sample of interest may be a nucleic acid sample, e.g., a DNA or RNA sample. The light source 210 can emit a light beam with at least one wavelength to excite fluorophores on more than one base, so as to achieve the sequencing of multiple bases.

In the embodiments of the present application, the light modulation component 220 is an optical element that forms random structured light by controlling and adjusting the amplitude, phase, polarization state, frequency, intensity, propagation direction, or the like of a light wave. By using the light modulation component 220, a light beam from the light source 210 can be converted into a spot with highly uniform energy distribution. The wavelength, shape, and contour of the spot may all be customized, and the shape may be any shape such as a circle, a square, a straight line, a rectangle, a hexagon, or an ellipse.

The light modulation component 220 may be an optical element formed by a transmissive scattering medium. Certainly, the light modulation component may also be an optical element formed by other transmissive scattering media. In some embodiments, the light modulation component 220 is a light homogenizer. The light homogenizer is also known as a homogenizing optic, a laser homogenizer, a homogenizer, a laser homogenizing element, a uniform spot diffractive element, a laser homogenization diffractive optical element (laser homogenization DOE), a laser beam homogenizer, a beam homogenizer, or a laser diffuser. In one embodiment, the light modulation component 220 may be a light homogenizer. The light homogenizer converts the illumination light into a uniform spot of any shape, and the shape of the light homogenizer includes, but is not limited to, a circle, a rectangle, and the like. The size of the super-resolution field of view of a light homogenizer-based optical system depends on the field of view of the objective lens rather than the super-resolution imaging field of view. Compared to a grating-based optical element, the light homogenizer-based optical system can achieve different structured illuminations without mechanically rotating the grating, thus improving the efficiency of super-resolution imaging.

Referring again to FIG. 3, in some certain embodiments, the light modulation component 220 is arranged on the output optical path of the light source 210.

As such, the light modulation component 220 can modulate the illumination light into a uniform spot before the illumination light is reflected to the objective lens 110.

Specifically, the light modulation component 220 can be controlled to move relative to the aperture of the objective lens 110.

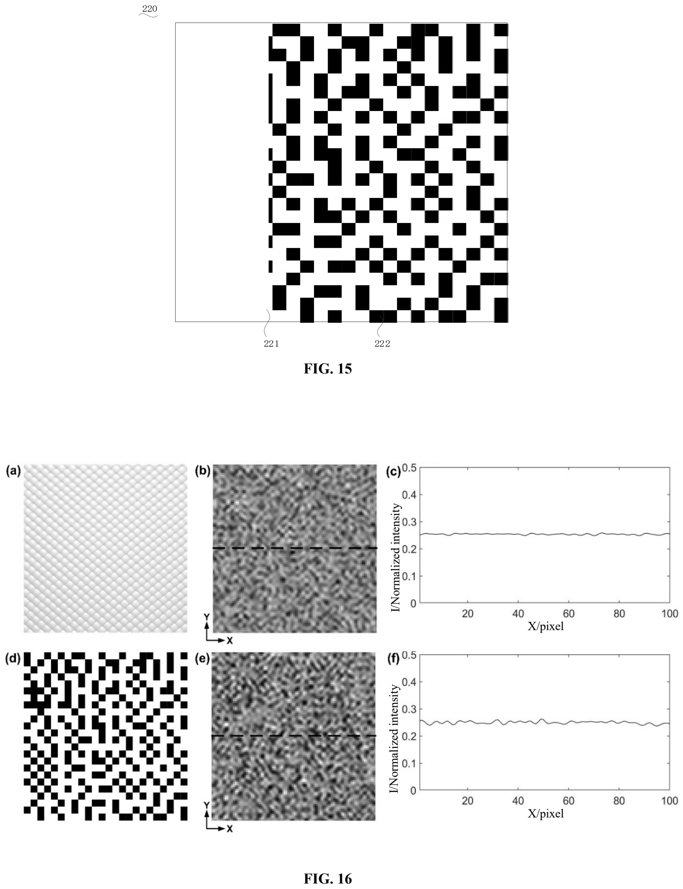

Referring to FIG. 15, in some certain embodiments, the light modulation component 220 is provided with a transmissive region 221 and an opaque region 222. The transmissive region 221 is configured for allowing the light emitted by the light source 210 to pass through to form the illumination light field, and the opaque region 222 is configured for preventing the light emitted by the light source 210 from passing through.

As such, by setting the area ratio of the transmissive region 221 to the opaque region 222, the illumination light field can be modulated.

In some embodiments, to achieve a better light field modulation effect, the area ratio of the transmissive region 221 to the opaque region 222 may be set between 1:3 and 3:1, including 1:3 and 3:1.

In some certain embodiments, the light modulation component 220 includes a sheet-like transmissive body, and at least one surface of the sheet-like transmissive body is provided with arranged opaque patterns to form the opaque region 222.

As such, a binarized light modulation component 220 may be obtained, thereby achieving a better light field modulation effect.

It will be appreciated that binarization can be simply interpreted as that "1" denotes the transmissive region 221, while "0" denotes the opaque region 222, and "0" and "1" thus denote the binarization. Specifically, the arranged opaque patterns may be provided on only one surface of the sheet-like transmissive body, i.e., the surface proximal to the light source 210 or the surface distal to the light source 210, or may be provided on both the surface proximal to the light source 210 and the surface distal to the light source 210. However, in any arrangement, the arranged opaque patterns can form the transmissive region 221 and the opaque region 222 on the sheet-like transmissive body, thereby further providing the binarized light modulation component 220.

In some certain embodiments, the opaque pattern is a patterned film layer formed by an opaque material having a light transmittance of less than or equal to 70%.

As such, the opaque region 222 formed by the opaque material having a light transmittance of less than or equal to 70% can effectively prevent the light emitted by the light source 210 from passing through.

In one embodiment of the present application, the surface of the sheet-like transmissive body may be plated with metal Cr. The part plated with Cr is the opaque region 222, and the part not plated with Cr is the transmissive region 221. Alternatively, the entire surface of the sheet-like transmissive body may be plated with Cr to form the opaque region 222 and then etched to form the transmissive region 221, such that the "transmissive region 221" and the "opaque region 222" each occupy a portion of the area.

Referring to FIG. 16, for conventional structured fringe illumination, the intensity of the excitation light satisfies the cosine distribution, i.e.,

$$I(r) = I_0 \left[ 1 + m \cdot \cos\left( 2\pi p \cdot r + \phi \right) \right] \dots\dots \text{formula (3)}$$

where $I_0$ denotes the average intensity of the structured illumination light field, m denotes the modulation depth, p denotes the illumination fringe frequency, $\emptyset$ denotes the fringe phase, and r denotes the position vector in a two-dimensional space. A higher modulation depth m indicates a better super-resolution effect. The illumination is typically generated by using a microlens array (FIG. 16a), a diffractive optical element, a transmissive scattering medium, and the like. The illumination light field generated by such a method has a low "modulation depth" (FIGs. 16b and c), and it is difficult to solve the illumination light field by using a super-resolution algorithm. Consequently, it is difficult to acquire a high-quality super-resolution reconstruction effect. The present application presents the use of a binarized light homogenizer. Patterns are arranged on the surface of the light homogenizer, with the area of the "transmissive region 221" and the area of the "opaque region 222" each accounting for 50% in general. Thus, the acquired "modulation depth" of the illumination light field is elevated (FIGs. 16d and e), thereby helping achieve a high-quality super-resolution reconstruction effect. It can be derived from formula (3):

$$\begin{cases} I_{max} = I_0 \cdot (1 + m) \\ I_{min} = I_0 \cdot (1 - m) \end{cases} \dots\dots \text{formula (4)}$$

The modulation depth can be acquired from the solution of formula (4):

$$m = \frac{I_{max} - I_{min}}{I_{max} + I_{min}} \dots\dots \text{formula (5)}$$

FIG. 16c shows the illumination light intensity distribution of a certain row (indicated by the black dashed line) in FIG. 16b. The maximum value and the minimum value of the normalized light intensity are 0.2586 and 0.2482, respectively. Thus, the modulation depth is: m = 0.0205. FIG. 16f shows the illumination light intensity distribution of a certain row (indicated by the black dashed line) in FIG. 16e. The maximum value and the minimum value of the normalized light intensity are 0.2626 and 0.2365, respectively. Thus, the modulation depth is: m = 0.0523, suggesting an increase of the modulation depth to

$$\frac{0.0523}{0.0205} \cdot 100\% = 255.12\%$$

As one embodiment of the present application, to achieve a better light field modulation effect, the arranged patterns satisfy a/K ≥ r, where a denotes the minimum unit size of the arranged patterns in microns; K denotes the reduction factor of the light beam of the illumination optical path after passing through the objective lens 110; and r denotes the diffraction-limited resolution of the objective lens 110.

Through the arranged opaque patterns, the light modulation component 220 generates fixed illumination to modulate the illumination light field. As the light modulation component 220 moves relative to the aperture of the objective lens 110, the illumination pattern obtained after the light source 210 passes through the light modulation component 220 varies, thus generating new illumination. Therefore, the illumination in the present application includes changes brought by the patterns and overlaps brought by the relative movement between the light modulation component 220 and the aperture of the objective lens 110, which better achieves the modulation of the illumination light field.

Referring to FIG. 3, in some certain embodiments, the illumination optical path assembly 200 includes an illumination lens set 230 arranged between the light source 210 and the light modulation component 220 and configured for expanding the illumination light. As such, through the beam expansion, the illumination light is converted into a uniform and magnified collimated light beam.

Specifically, the illumination lens set 230 may be a beam expander. The beam expander is configured for expanding the diameter of a light beam and reducing the divergence angle of the light beam, so as to achieve long-distance illumination and focusing.

Referring again to FIG. 3, in some certain embodiments, the illumination optical path assembly 200 includes an illumination mirror 240 located on the side of the light modulation component 220 distal to the light source 210 and configured for reflecting the illumination light field to the objective lens 110.

As such, the illumination mirror 240 reflects the illumination light field to the objective lens 110, such that the illumination light field irradiates the sample of interest after passing through the objective lens 110 to excite the sample of interest to generate fluorescence with various wavelengths.

Specifically, the illumination mirror 240 is located on the output optical path of the light source 210 and arranged at an included angle of 45° relative to the light of the light source 210.

As shown in FIG. 5, in some embodiments, two light sources 210 of different wavelengths are employed to excite fluorophores on different bases, thereby achieving the sequencing of four types of bases A, T, G, and C. Specifically, a beam combiner component 250 may be used to combine the beams for the two light sources 210 of different wavelengths, and then after passing through the illumination lens set 230, the light beam is modulated by the light modulation component 220 to generate an illumination light field. As such, the imaging optical path assembly 100 can acquire a fluorescence signal in the illumination condition to form an illumination-scanning widefield image. The beam combiner component 250 may be a dichroic mirror that combines the beams from the light sources 210 of different wavelengths into an illumination light beam.

Referring to FIG. 1, in some certain embodiments, the driving component 300 is configured for driving the light modulation component 220 and/or the sample to move relative to the objective lens 110, so as to update a pattern of the illumination light field located on the sample.

As such, the driving component 300 drives the light modulation component 220 to move relative to the sample and the aperture of the objective lens 110, such that the light modulation component 220 can perform step-by-step scanning in a single field of view according to a specific rule, with a widefield image being acquired in each scanning step. As such, a plurality of widefield images in the illumination light field may be acquired in a plurality of scanning steps, thereby enabling the optical imaging assembly 120 to acquire a plurality of illumination-scanning widefield images.

Specifically, the driving component 300 may drive the light modulation component 220 to move relative to the objective lens 110, or may also drive the sample to move relative to the objective lens 110.

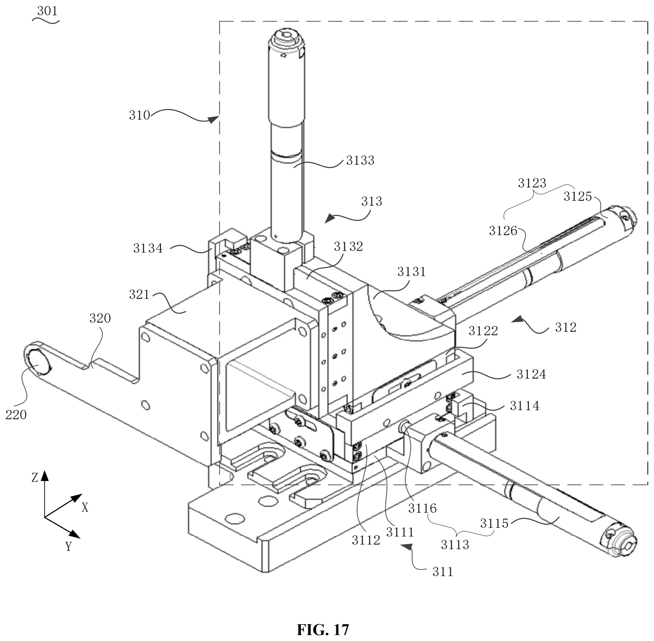

Referring to FIG. 17, in some certain embodiments, the driving component 300 includes a first driving component 301. The first driving component 301 includes a driving structure 310 and a mounting member 320 connected to the driving structure 310. The light modulation component 220 is fixed on the mounting member 320. The driving structure 310 drives, through the mounting member 320, the light modulation component 220 to move relative to the objective lens 110.

As such, as the driving structure 310 moves, the light modulation component 220 also moves relative to the aperture of the objective lens 110, thereby updating the pattern of the illumination light field.

Specifically, the mounting member 320 may be of a circular or rectangular plate-like structure. One end of the mounting member 320 is connected to the driving structure 310 through an extension plate 321 and moves along with the driving structure 310, and the other end secures the light modulation component 220 and drives the light modulation component 220 to move. The extension plate 321 can adjust the height of the light modulation component 220 mounted on the driving structure 310 according to actual situations in practical applications, suggesting better applicability.

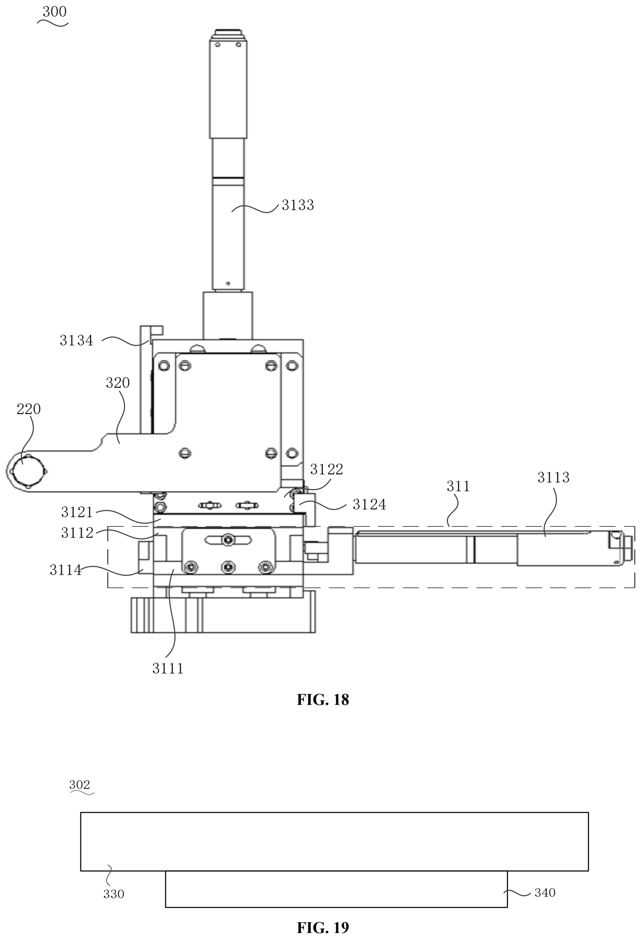

Referring again to FIGs. 17 and 18, in some certain embodiments, the driving structure 310 includes a plurality of sliding assemblies. The plurality of sliding assemblies are connected in sequence. Each of the sliding assemblies is configured for driving the mounting member 320 to move in at least one of a first direction Y, a second direction X, and a third direction Z, and the first direction, the second direction, and the third direction are perpendicular to each other. The mounting member 320 is connected to one of the sliding assemblies.

As such, through the movement of the mounting member 320 in the first direction Y, the second direction X, and the third direction Z, the light modulation component 220 can be moved in the first direction Y, the second direction X, and the third direction Z.

Specifically, the mounting member 320 may move in one of the first direction Y, the second direction X, and the third direction Z, may move in two of the first direction Y, the second direction X, and the third direction Z, or may move in three of the first direction Y, the second direction X, and the third direction Z. Referring again to FIG. 17, in some certain embodiments, the sliding assembly includes a first sliding assembly 311, a second sliding assembly 312, and a third sliding assembly 313. The second sliding assembly 312 is connected to the first sliding assembly 311 and the third sliding assembly 313, and the mounting member 320 is connected to the third sliding assembly 313. The first sliding assembly 311 is configured for driving the second sliding assembly 312 and the third sliding assembly 313 to move in the first direction Y as a whole, so as to drive the light modulation component 220 to move in the first direction Y. The second sliding assembly 312 is configured for driving the third sliding assembly 313 to move in the second direction X, so as to drive the light modulation component 220 to move in the second direction X. The third sliding assembly 313 is fixed to the mounting member 320 and configured for driving the mounting member 320 to move in the third direction Z, so as to drive the light modulation component 220 to move in the third direction Z.

As such, a tiny movement of the light modulation component 220 can be achieved, thereby enabling the precise modulation of the illumination light field.

Specifically, the first sliding assembly 311 is perpendicular to the second sliding assembly 312 and the third sliding assembly 313 in the first direction Y, the second sliding assembly 312 is perpendicular to the first sliding assembly 311 and the third sliding assembly 313 in the second direction X, and the third sliding assembly 313 is perpendicular to the first sliding assembly 311 and the second sliding assembly 312 in the third direction Z.

Referring to FIG. 18, in some certain embodiments, the first sliding assembly 311 includes a first fixed member 3111, a first sliding member 3112, and a first driving member 3113. The first sliding member 3112 is slidably arranged on the first fixed member 3111. The first driving member 3113 is fixed to the first fixed member 3111 and configured for driving the first sliding member 3112 to move in the first direction Y relative to the first fixed member 3111. The first sliding member 3112 is fixed to the second sliding assembly 312.

As such, the second sliding assembly 312 can be moved in the first direction Y, thereby enabling the movement of the light modulation component 220 in the first direction Y.

Specifically, a guide rail (not shown) may be arranged on the first fixed member 3111, and the first sliding member 3112 may be slidably arranged on the guide rail of the first fixed member 3111. A first limiting plate 3114 is arranged on a side edge of the first fixed member 3111 parallel to the guide rail, and the first limiting plate 3114 is configured for limiting the travel of movement of the first sliding assembly 311 in the first direction Y. Since the movement range of the light modulation component 220 is relatively small, to prevent an excessive movement range, the first limiting plate 3114 is arranged on the first fixed member 3111, so as to ensure that the maximum movement range of the light modulation component 220 is within the movement range, thereby enabling the precise regulation of the illumination light field.

In some certain embodiments, the first driving member 3113 includes a first motor 3115 and a first driving shaft 3116. The first motor 3115 is fixed to the first fixed member 3111 and located on one side of the first sliding member 3112. One end of the first driving shaft 3116 is connected to the first motor 3115, and the other end is connected to the first sliding member 3112. The first motor 3115 controls the driving shaft to extend and retract, so as to drive the first sliding member 3112 to move in the first direction Y relative to the first fixed member 3111.

As such, the second sliding assembly 312 can be moved in the first direction Y, thereby enabling the movement of the light modulation component 220 in the first direction Y.

Specifically, the first sliding member 3112 and the first fixed member 3111 may be connected by an elastic member therebetween. The elastic member may be a spring. When a relative displacement is generated between the first sliding member 3112 and the first fixed member 3111 and the driving shaft retracts, the elastic member repositions the first sliding member 3112.

Referring again to FIG. 17, in some certain embodiments, the second sliding assembly 312 includes a second fixed member 3121, a second sliding member 3122, and a second driving member 3123. The second sliding member 3122 is slidably arranged on the second fixed member 3121. The second driving member 3123 is fixed to the second fixed member 3121 and configured for driving the second sliding member 3122 to move in the second direction X relative to the second fixed member 3121. The second fixed member 3121 is fixed to the first sliding member 3112, and the second sliding member 3122 is fixed to the third sliding assembly 313.

As such, the third sliding assembly 313 can be moved in the second direction X, thereby enabling the movement of the light modulation component 220 in the second direction X.

Specifically, a guide rail may be arranged on the second fixed member 3121, and the second sliding member 3122 may be slidably arranged on the guide rail of the second fixed member 3121. A second limiting plate 3124 is arranged on a side edge of the second fixed member 3121 parallel to the guide rail, and the second limiting plate 3124 is configured for limiting the travel of movement of the second sliding assembly 312 in the second direction X. Since the movement range of the light modulation component 220 is relatively small, to prevent an excessive movement range, the second limiting plate 3124 is arranged on the second fixed member 3121, so as to ensure that the maximum movement range of the light modulation component 220 is within the movement range, thereby enabling the precise regulation of the illumination light field.

In some certain embodiments, the second driving member 3123 includes a second motor 3125 and a second driving shaft 3126. The second motor 3125 is fixed to the second fixed member 3121 and located on one side of the second sliding member 3122. One end of the second driving shaft 3126 is connected to the second motor 3125, and the other end is connected to the second sliding member 3122. The second motor 3125 controls the driving shaft to extend and retract, so as to drive the second sliding member 3122 to move in the second direction X relative to the second fixed member 3121.

As such, the third sliding assembly 313 can be moved in the second direction X, thereby enabling the movement of the light modulation component 220 in the second direction X.

Specifically, the second sliding member 3122 and the second fixed member 3121 may be connected by an elastic member therebetween. The elastic member may be a spring. When a relative displacement is generated between the second sliding member 3122 and the second fixed member 3121 and the driving shaft retracts, the elastic member repositions the second sliding member 3122.

Referring again to FIG. 17, in some certain embodiments, the third sliding assembly 313 includes a third fixed member 3131, a third sliding member 3132, and a third driving member 3133. The third sliding member 3132 is slidably arranged on the third fixed member 3131. The third driving member 3133 is fixed to the third fixed member 3131 and configured for driving the third sliding member 3132 to move in the third direction Z relative to the third fixed member 3131. The third fixed member 3131 is fixed to the second sliding member 3122, and the third sliding member 3132 is fixed to the mounting member 320.

As such, the mounting member 320 can be moved in the third direction Z, thereby enabling the movement of the light modulation component 220 in the third direction Z.

Specifically, a guide rail is arranged on the third fixed member 3131, and the third sliding member 3132 is slidably arranged on the guide rail of the third fixed member 3131. A third limiting plate 3134 is arranged on a side edge of the third fixed member 3131 parallel to the guide rail, and the third limiting plate 3134 is configured for limiting the travel of movement of the third sliding assembly 313 in the third direction Z. Since the movement range of the light modulation component 220 is relatively small, to prevent an excessive movement range, the third limiting plate 3134 is arranged on the third fixed member 3131, so as to ensure that the maximum movement range of the light modulation component 220 is within the movement range, thereby enabling the precise regulation of the illumination light field.

In some certain embodiments, the third driving member 3133 comprises a third motor (not shown) and a third driving shaft (not shown). The third motor is fixed to the third fixed member 3131 and located on one side of the third sliding member 3132. One end of the third driving shaft is connected to the third motor, and the other end is connected to the third sliding member 3132. The third motor controls the driving shaft to extend and retract, so as to drive the third sliding member 3132 to move in the third direction Z relative to the third fixed member 3131.

As such, the mounting member 320 can be moved in the third direction Z, thereby enabling the movement of the light modulation component 220 in the third direction Z.

Specifically, the third sliding member 3132 and the third fixed member 3131 may be connected by an elastic member therebetween. The elastic member may be a spring. When a relative displacement is generated between the third sliding member 3132 and the third fixed member 3131 and the driving shaft retracts, the elastic member repositions the third sliding member 3132.

In some certain embodiments, the driving component 300 includes a second driving component 302. The second driving component 302 is configured for driving the sample to move relative to the objective lens 110.

As such, on the one hand, the translation of the sample can be achieved through the second driving component 302, thereby enabling the acquisition of optical signals at different regions of the sample surface; on the other hand, the second driving component 302 allows the relative movement between the sample and the aperture of the objective lens 110, thereby updating the pattern of the illumination light field. Referring to FIG. 19, in some certain embodiments, the second driving component 302 is configured for driving the sample to move relative to the objective lens 110 in a plane perpendicular to an optical axis 111 of the objective lens 110.

Specifically, the second driving component 302 may include a carrying platform 330 and a two-dimensional driving assembly 340 arranged under the carrying platform 330. The carrying platform 330 can carry the sample, and the two-dimensional driving assembly 340 can drive the carrying platform 330 to move in a plane perpendicular to the optical axis 111 of the objective lens 110, thereby driving the sample to move relative to the objective lens 110 in a plane perpendicular to the optical axis 111 of the objective lens 110. The two-dimensional driving assembly 340 may include two sliding assemblies. One sliding assembly drives the carrying platform 330 to move in one direction in the plane perpendicular to the optical axis 111 of the objective lens 110, and the other driving assembly drives the carrying platform 330 to move in another direction in the plane perpendicular to the optical axis 111 of the objective lens 110. The two sliding assemblies, when cooperating with each other, can drive the carrying platform 330 to move in a plane perpendicular to the optical axis 111 of the objective lens 110.

The specific structures of the two sliding assemblies in the two-dimensional driving assembly 340 may be similar to those of the first sliding assembly 311 and the second sliding assembly 312 discussed above. For example, the sliding assembly in the two-dimensional driving assembly 340 may be provided with a fixed member, a sliding member, and a driving member. The driving member may drive the sliding member to move relative to the fixed member, thereby driving the carrying platform 330 to move. As such, the second driving component 302 can achieve the movement of the sample, thereby enabling the precise modulation for the illumination light field.

Referring to FIG. 4, in some certain embodiments, the optical system 1000 includes a focusing assembly 400. During the gene sequencing process, the focusing assembly 400 monitors the variation of the object distance in real time, so as to ensure that the optical system 1000 always focuses sharply, thereby ensuring the accuracy of acquired images. The focusing assembly 400 includes a second light source 410 and a focusing sensor 420. The second light source 410 is configured for projecting a light beam emitted by the second light source 410 onto a sample of interest through the objective lens 110. The focusing sensor 420 is configured for receiving a light beam reflected by the sample of interest and collimated by the objective lens 110 and converting an optical signal into an electric signal, so as to enable the objective lens 110 to move according to the electric signal and enable the plane where the sample of interest is located to be positioned at the focal plane of the objective lens 110.

The focusing assembly 400 is mainly configured for marking the object distance after the objective lens 110 finishes the focusing, monitoring the variation of the object distance in real time during the gene sequencing process, and correcting the variation, so as to ensure that the system always focuses sharply, thereby ensuring the accuracy of the images acquired by a detection camera and the acquisition of sharp images within the depth of focus range.

The focusing sensor 420 is configured for receiving a light beam reflected by the sample of interest and collimated by the objective lens 110. The focusing sensor 420 determines the distance between the plane where the sample of interest is located and the focal plane of the objective lens 110 according to the light beam, and converts an optical signal into an electrical signal. The electrical signal is configured for characterizing the distance between the plane where the sample of interest is located and the focal plane of the objective lens 110. By adjusting the plane where the sample of interest is located according to the electrical signal, the plane where the sample of interest is located can be positioned at the focal plane of the objective lens 110.

A fourth dichroic mirror 133 is arranged on the optical axis 111 of the objective lens 110 and configured for reflecting a light beam emitted by the second light source 410, so as to project the light beam emitted by the second light source 410 onto the sample of interest sequentially through the objective lens 110 and reflecting the light beam reflected by the sample of interest to the focusing sensor 420.

The gene sequencing device according to the embodiments of the present application includes an optical system 1000. The sequencing device according to embodiments of the present application includes, but is not limited to, devices having sequencing functionality such as gene sequencing devices and nucleic acid sequencing devices, while other systems or devices manufactured as per the same principle are also applicable to the embodiments of the present application.

In summary, in one embodiment of the present application, the optical system 1000 according to the embodiments of the present application includes the light source 210, the light modulation component 220, the objective lens 110, the optical imaging assembly 120, and the driving component 300. The light modulation component 220 is configured for transmitting and modulating an illumination light generated by the light source 210 to form an illumination light field. The objective lens 110 is arranged on an optical path of the illumination light field and configured for projecting the illumination light field onto a sample and receiving a fluorescence signal generated by the sample. The sample is arranged on the optical path of the illumination light field and located on the side of the objective lens 110 distal to the light modulation component 220. The optical imaging assembly 120 is configured for receiving an optical signal and forming an image. The driving component 300 is configured for driving the light modulation component 220 and/or the sample to move relative to the objective lens 110, so as to update a pattern of the illumination light field located on the sample.

By using the optical system 1000 of the solution, the driving component 300 drives the light modulation component 220 and/or the sample to move relative to the objective lens 110, so as to update the pattern of the illumination light field located on the sample, such that the pattern of the illumination light field located on the sample can be precisely updated. This effectively improves the imaging efficiency of the optical system 1000 and reduces the complexity of the system and the manufacturing costs.

In the description of the specification, references to the terms such as "one embodiment", "certain embodiments", "schematic embodiments", "examples", "specific examples", "some examples", or the like, mean that the particular features, structures, materials or characteristics contained in the embodiments or examples are included in at least one embodiment or example of the present application. In the specification, the schematic description of the above terms does not necessarily refer to the same embodiment or example. Moreover, the specific feature, structure, material, or characteristic described may be combined in any one or more embodiments or examples in any appropriate manner.

Although the embodiments of the present application have been illustrated and described, it will be appreciated by those of ordinary skill in the art that various changes, modifications, replacements, and variations can be made to these embodiments without departing from the principle and spirit of the present application, and the scope of the present application is defined by the claims and equivalents thereof.

## Claims

1. An optical system, comprising:

   a light source;
   a light modulation component, configured for transmitting and modulating an illumination light generated by the light source to form an illumination light field;

an objective lens, arranged on an optical path of the illumination light field and configured for projecting the illumination light field onto a sample and receiving an optical signal generated by the sample, wherein the sample is arranged on the optical path of the illumination light field and is located on the side of the objective lens distal to the light modulation component;

an optical imaging assembly, configured for receiving the optical signal and forming an image; and

a driving component, configured for driving the light modulation component and/or the sample to move relative to the objective lens, so as to update a pattern of the illumination light field located on the sample.

2. The optical system according to claim 1, wherein the light modulation component is arranged on an output optical path of the light source.

3. The optical system according to claim 1 or 2, wherein the driving component comprises a first driving component; the first driving component comprises a driving structure and a mounting member connected to the driving structure; the light modulation component is fixed on the mounting member; the driving structure drives, through the mounting member, the light modulation component to move relative to the objective lens.

4. The optical system according to claim 3, wherein the driving structure comprises a plurality of sliding assemblies; the plurality of sliding assemblies are connected in sequence; each of the sliding assemblies is configured for driving the mounting member to move in at least one of a first direction, a second direction, and a third direction, and the first direction, the second direction, and the third direction are perpendicular to each other; the mounting member is connected to one of the sliding assemblies.

5. The optical system according to claim 4, wherein the sliding assembly comprises a first sliding assembly, a second sliding assembly, and a third sliding assembly; the second sliding assembly is connected to the first sliding assembly and the third sliding assembly, and the mounting member is connected to the third sliding assembly; the first sliding assembly is configured for driving the second sliding assembly and the third sliding assembly to move in the first direction as a whole, so as to drive the light modulation component to move in the first direction; the second sliding assembly is configured for driving the third sliding assembly to move in the second direction, so as to drive the light modulation component to move in the second direction; the third sliding assembly is fixed to the mounting member and configured for driving the mounting member to move in the third direction, so as to drive the light modulation component to move in the third direction.

6. The optical system according to claim 5, wherein the first sliding assembly comprises a first fixed member, a first sliding member, and a first driving member; the first sliding member is slidably arranged on the first fixed member; the first driving member is fixed to the first fixed member and configured for driving the first sliding member to move in the first direction relative to the first fixed member; the first sliding member is fixed to the second sliding assembly.

7. The optical system according to claim 6, wherein the first driving member comprises a first motor and a first driving shaft; the first motor is fixed to the first fixed member and located on one side of the first sliding member; one end of the first driving shaft is connected to the first motor, and the other end is connected to the first sliding member; the first motor controls the driving shaft to extend and retract, so as to drive the first sliding member to move in the first direction relative to the first fixed member.

8. The optical system according to any one of claims 5 to 7, wherein the second sliding assembly comprises a second fixed member, a second sliding member, and a second driving member; the second sliding member is slidably arranged on the second fixed member; the second driving member is fixed to the second fixed member and configured for driving the second sliding member to move in the second direction relative to the second fixed member; the second fixed member is fixed to the first sliding member, and the second sliding member is fixed to the third sliding assembly.

9. The optical system according to claim 8, wherein the second driving member comprises a second motor and a second driving shaft; the second motor is fixed to the second fixed member and located on one side of the second sliding member; one end of the second driving shaft is connected to the second motor, and the other end is connected to the second sliding member; the second motor controls the driving shaft to extend and retract, so as to drive the second sliding member to move in the second direction relative to the second fixed member.

10. The optical system according to any one of claims 5 to 9, wherein the third sliding assembly comprises a third fixed member, a third sliding member, and a third driving member; the third sliding member is slidably arranged on the third fixed member; the third driving member is fixed to the third fixed member and configured for driving the third sliding member to move in the third direction relative to the third fixed member; the third fixed member is fixed to the second sliding member, and the third sliding member is fixed to the mounting member.

11. The optical system according to claim 10, wherein the third driving member comprises a third motor and a third driving shaft; the third motor is fixed to the third fixed member and located on one side of the third sliding member; one end of the third driving shaft is connected to the third motor, and the other end is connected to the third sliding member; the third motor controls the driving shaft to extend and retract, so as to drive the third sliding member to move in the third direction relative to the third fixed member.

12. The optical system according to claim 3, wherein the driving component comprises a second driving component, and the second driving component is configured for driving the sample to move relative to the objective lens.

13. The optical system according to claim 12, wherein the second driving component is configured for driving the sample to move relative to the objective lens in a plane perpendicular to the optical axis of the objective lens.

14. The optical system according to any one of claims 1 to 13, wherein the light modulation component is provided with a transmissive region and an opaque region; the transmissive region is configured for allowing the light emitted by the light source to pass through to form the illumination light field, and the opaque region is configured for preventing the light emitted by the light source from passing through.

15. The optical system according to claim 14, wherein the light modulation component comprises a sheet-like transmissive body, and at least one surface of the sheet-like transmissive body is provided with randomly arranged opaque patterns to form the opaque region.

16. The optical system according to claim 15, wherein the opaque pattern is a patterned film layer formed by an opaque material having a light transmittance of less than or equal to 70%.

17. The optical system according to any one of claims 1 to 16, further comprising a splitter mechanism arranged between the objective lens and the optical imaging assembly, wherein the splitter mechanism is configured for splitting the optical signal into different light beams; the optical imaging assembly comprises a plurality of cameras, and each of the cameras is configured for receiving one of the light beams and generating an image.

18. The optical system according to claim 17, wherein the splitter mechanism is configured for splitting the optical signal into a first light beam and a second light beam; the optical imaging assembly comprises a first camera and a second camera spaced apart from the first camera; the first camera is configured for receiving the first light beam and generating an image, and the second camera is configured for receiving the second light beam and generating an image.

19. The optical system according to claim 18, wherein the splitter mechanism comprises a first dichroic mirror, and the first dichroic mirror is configured for transmitting the optical signal to form the first light beam and reflecting the optical signal to form the second light beam.

20. The optical system according to claim 19, wherein the first dichroic mirror is arranged aside from the optical axis of the objective lens.

21. The optical system according to claim 19 or 20, wherein the splitter mechanism further comprises a mirror arranged on the optical axis of the objective lens, and the mirror is arranged between the objective lens and the first dichroic mirror and configured for reflecting the optical signal to the first dichroic mirror.

22. The optical system according to claim 21, wherein the mirror is arranged parallel or perpendicular to the first dichroic mirror.

23. The optical system according to claim 21 or 22, further comprising an illumination dichroic mirror arranged on the optical axis of the objective lens, wherein the illumination dichroic mirror is arranged between the mirror and the objective lens and configured for reflecting the illumination light field to the objective lens and transmitting the optical signal.

24. The optical system according to claim 17, wherein the splitter mechanism is configured for splitting the optical signal into a first light beam, a second light beam, a third light beam, and a fourth light beam; the optical imaging assembly comprises a first camera, a second camera, a third camera, and a fourth camera; the first camera is configured for receiving the first light beam and generating an image, the second camera is configured for receiving the second light beam and generating an image, the third camera is configured for receiving the third light beam and generating an image, and the fourth camera is configured for receiving the fourth light beam and generating an image.

25. The optical system according to claim 24, wherein the splitter mechanism comprises a first splitter mechanism, a second splitter mechanism, a third splitter mechanism, and a fourth splitter mechanism;

the first splitter mechanism splits the optical signal to form a first mixed light beam and the fourth light beam; the first splitter mechanism is further configured for splitting a portion of the first mixed light beam to form the first light beam, converging the first light beam to the first camera, and transmitting another portion of the first mixed light beam as a second mixed light beam to the second splitter mechanism;
the second splitter mechanism is configured for receiving the second mixed light beam, splitting the second mixed light beam to form the second light beam and the third light beam, converging the second light beam to the second camera, and transmitting the third light beam to the third splitter mechanism;
the third splitter mechanism is configured for converging the third light beam to the third camera;
the fourth splitter mechanism receives the fourth light beam formed by the first splitter mechanism and converges the fourth light beam to the fourth camera.

26. The optical system according to claim 25, wherein the first splitter mechanism comprises a first dichroic mirror and a second dichroic mirror; the first dichroic mirror is arranged on the optical axis of the objective lens, placed obliquely relative to the optical axis of the objective lens, and configured for reflecting a light from the objective lens to form the first mixed light beam and transmitting the light from the objective lens to form the fourth light beam;
the second dichroic mirror is spaced apart from the first dichroic mirror and configured for reflecting a portion of the first mixed light beam to form the first light beam and directing the first light beam to the first camera, and transmitting another portion of the first mixed light beam to form the second mixed light beam.

27. The optical system according to claim 26, wherein the included angle between the first dichroic mirror and the second dichroic mirror is 90° ± 15°.

28. The optical system according to claim 27, wherein the first dichroic mirror is arranged perpendicular to the second dichroic mirror.

29. The optical system according to claim 26, wherein the first splitter mechanism further comprises a first mirror; the first mirror is spaced apart from the second dichroic mirror and configured for reflecting a portion of the first light beam from the second dichroic mirror to the first camera.

30. The optical system according to claim 28, wherein the second splitter mechanism comprises a third dichroic mirror; the third dichroic mirror is spaced apart from the second dichroic mirror, arranged on the side of the second dichroic mirror distal to the first dichroic mirror, and configured for reflecting a portion of the second mixed light beam to form the second light beam and directing the second light beam to the second camera, and transmitting another portion of the second mixed light beam to form the third light beam.

31. The optical system according to claim 30, wherein the second dichroic mirror is substantially parallel to the third dichroic mirror.

32. The optical system according to claim 30, wherein the included angle between the third dichroic mirror and the second dichroic mirror is 90° ± 15°.

33. The optical system according to claim 32, wherein the third dichroic mirror is arranged perpendicular to the second dichroic mirror.

34. The optical system according to claim 30, wherein the third splitter mechanism comprises a second mirror; the second mirror is spaced apart from the third dichroic mirror, arranged on the side of the third dichroic mirror distal to the second dichroic mirror, and configured for reflecting the third light beam to the third camera.

35. The optical system according to claim 34, wherein the second mirror is arranged parallel or perpendicular to the third dichroic mirror.

36. The optical system according to claim 26, wherein the fourth splitter mechanism comprises a third mirror; the third mirror is arranged on the optical axis of the objective lens and obliquely relative to the optical axis of the objective lens, located on the side of the first dichroic mirror distal to the objective lens, and configured for reflecting the fourth light beam to the fourth camera.

37. The optical system according to claim 36, wherein the first camera, the second camera, and the third camera are located on a first plane; the first plane intersects the optical axis of the objective lens; the fourth camera is located on one side of the first plane.

38. The optical system according to claim 37, wherein the first plane is perpendicular to the optical axis of the objective lens.

39. The optical system according to claim 38, wherein the fourth camera is located on a second plane parallel to the first plane.

40. The optical system according to any one of claims 37 to 39, wherein the normal direction of the imaging surface of the first camera and the normal direction of the imaging surface of the fourth camera are identical or opposite.

41. The optical system according to any one of claims 37 to 39, wherein the normal direction of the imaging surface of the second camera and the normal direction of the imaging surface of the third camera are identical.

42. The optical system according to any one of claims 37 to 41, wherein the distance from the center of the fourth camera to the optical axis of the objective lens is less than the distance from the center of the first camera to the optical axis of the objective lens; and/or,

the distance from the center of the second camera to the optical axis of the objective lens is less than the distance from the center of the third camera to the optical axis of the objective lens; and/or,
the first camera and the second camera are separately located on two sides of a third plane; the third plane passes through the optical axis of the objective lens and the center of the fourth camera.

43. The optical system according to claim 24, wherein the splitter mechanism comprises a first splitter mechanism, a second splitter mechanism, and a third splitter mechanism;

the first splitter mechanism splits the optical signal to form a first mixed light beam and a second mixed light beam; the second splitter mechanism is configured for splitting the first mixed light beam to form the first light beam and the second light beam, and converging the first light beam to the first camera and the second light beam to the second camera;
the third splitter mechanism is configured for splitting the second mixed light beam to form the third light beam and the fourth light beam, and converging the third light beam to the third camera and the fourth light beam to the fourth camera.

44. The optical system according to claim 43, wherein a plurality of light sources are provided; different light sources emit lights of different wavebands, and the lights of different wavebands excite optical signals of different wavebands; the first camera, the second camera, the third camera, and the fourth camera are configured for generating an image according to the optical signals of different wavebands.

45. The optical system according to claim 43 or 44, wherein the first splitter mechanism comprises a first dichroic mirror, the second splitter mechanism comprises a second dichroic mirror, and the third splitter mechanism comprises a third dichroic mirror;

the first dichroic mirror splits the optical signal to form a first mixed light beam and a second mixed light beam; the second dichroic mirror is configured for splitting the first mixed light beam to form the first light beam and the second light beam, and converging the first light beam to the first camera and the second light beam to the second camera;
the third dichroic mirror is configured for splitting the second mixed light beam to form the third light beam and the fourth light beam, and converging the third light beam to the third camera and the fourth light beam to the fourth camera.

46. The optical system according to claim 45, wherein the first dichroic mirror is parallel to the second dichroic mirror, and/or the first dichroic mirror is perpendicular to the third dichroic mirror.

47. The optical system according to claim 46, wherein the first dichroic mirror is arranged aside from the optical axis of the objective lens.

48. The optical system according to any one of claims 17 to 47, further comprising a tube lens arranged on the object-side optical path of the camera and configured for generating an image on the camera.

49. The optical system according to claim 48, wherein the tube lens is located between the camera and the splitter mechanism; or the tube lens is located on the internal optical path of the splitter mechanism.

50. The optical system according to claim 48 or 49, wherein the tube lens sequentially comprises:

a first lens, having a negative refractive power;
a second lens, having a positive refractive power;
a third lens, having a positive refractive power; and
a fourth lens, having a negative refractive power;
the object-side surfaces and the image-side surfaces of the first lens, the second lens, the third lens, and the fourth lens are all spherical surfaces; the object-side surface of one lens and the image-side surface of the other lens in every pair of adjacent lenses are arranged opposite.

51. The optical system according to claim 50, wherein the tube lens satisfies the following relational formula:

$$\frac{\Delta l'_{FC}}{\beta^2} \leq \frac{DOF}{6}$$

wherein denotes the axial chromatic aberration of the tube lens, denotes the magnification factor of the tube lens, and DOF denotes the depth of field of the tube lens.

52. The optical system according to any one of claims 50 to 51, wherein the object-side surface of the first lens is a first convex surface at the optical axis, and the image-side surface of the first lens is a first concave surface at the optical axis;
the first convex surface has a curvature radius of 100 mm to 200 mm; and/or the image-side surface of the first concave surface has a curvature radius of 20 mm to 40 mm at the optical axis.

53. The optical system according to claim 50 or 51, wherein the object-side surface of the second lens is a second convex surface at the optical axis, and the image-side surface of the second lens is a third convex surface at the optical axis; the object-side surface of the second lens has a curvature radius of 20 mm to 40 mm at the optical axis; and/or the image-side surface of the second lens has a curvature radius of -250 mm to -200 mm at the optical axis.

54. The optical system according to claim 50 or 51, wherein the object-side surface of the third lens is a fourth convex surface at the optical axis, and the image-side surface of the third lens is a second concave surface at the optical axis; the object-side surface of the third lens has a curvature radius of 20 mm to 40 mm at the optical axis; and/or the image-side surface of the third lens has a curvature radius of 400 mm to 500 mm at the optical axis.

55. The optical system according to claim 50 or 51, wherein the object-side surface of the fourth lens is a fifth convex surface at the optical axis, and the image-side surface of the fourth lens is a third concave surface at the optical axis; the object-side surface of the fourth lens has a curvature radius of 400 mm to 500 mm at the optical axis; and/or the image-side surface of the fourth lens has a curvature radius of 20 mm to 40 mm at the optical axis.

56. The optical system according to claim 50 or 51, wherein the object-side surface of the first lens is a first convex surface at the optical axis, and the image-side surface of the first lens is a first concave surface at the optical axis; the object-side surface of the second lens is a second convex surface at the optical axis, and the image-side surface of the second lens is a third convex surface at the optical axis; the object-side surface of the third lens is a fourth convex surface at the optical axis, and the image-side surface of the third lens is a second concave surface at the optical axis; the object-side surface of the fourth lens is a fifth convex surface at the optical axis, and the image-side surface of the fourth lens is a third concave surface at the optical axis;

the first convex surface has a curvature radius of 100 mm to 200 mm; and/or the image-side surface of the first concave surface has a curvature radius of 20 mm to 40 mm at the optical axis; and/or the object-side surface of the second lens has a curvature radius of 20 mm to 40 mm at the optical axis; and/or the image-side surface of the second lens has a curvature radius of -250 mm to -200 mm at the optical axis; and/or, the object-side surface of the third lens has a curvature radius of 20 mm to 40 mm at the optical axis; and/or the image-side surface of the third lens has a curvature radius of 400 mm to 500 mm at the optical axis; and/or, the object-side surface of the fourth lens has a curvature radius of 400 mm to 500 mm at the optical axis; and/or the image-side surface of the fourth lens has a curvature radius of 20 mm to 40 mm at the optical axis.

57. The optical system according to claim 50 or 51, wherein the first lens has a thickness of 2.0 mm to 3.0 mm at the optical axis; and/or,
the second lens has a thickness of 9 mm to 12 mm at the optical axis, the third lens has a thickness of 7 mm to 10 mm at the optical axis, and the fourth lens has a thickness of 2.0 mm to 3.0 mm at the optical axis.

58. The optical system according to any one of claims 50 to 57, wherein the first lens and the second lens are cemented to form a lens set, and the third lens and the fourth lens are cemented to form a lens set.

59. The optical system according to any one of claims 48 to 57, wherein the object-side field of view of the combination of the tube lens with the objective lens is greater than 1.0 mm.

60. The optical system according to any one of claims 48 to 59, wherein the tube lens satisfies the following relational formula:

$$120 \text{ mm} < f < 140 \text{ mm};$$

wherein f denotes the effective focal length of the tube lens.

61. The optical system according to any one of claims 48 to 60, wherein the tube lens has a back focal distance greater than or equal to 70 mm.

62. The optical system according to any one of claims 1 to 61, further comprising an illumination lens set arranged between the light source and the light modulation component and configured for expanding the illumination light.

63. The optical system according to claim 62, further comprising an illumination mirror located on the side of the light modulation component distal to the light source and configured for reflecting the illumination light field to the objective lens.

64. A gene sequencing device, comprising the optical system according to any one of claims 1 to 63.

1000

**FIG. 1**

**FIG. 2**

**FIG. 3**

**FIG. 4**

**FIG. 5**

**FIG. 6**

**FIG. 7**

**FIG. 8**

**FIG. 9**

**FIG. 10**

**FIG. 11**

**FIG. 12**

**FIG. 13**

**FIG. 14**

FIG. 15

FIG. 16

**FIG. 17**

300

3133

3134

320

220

3122

3121
3112

3124

311

3113

3114

3111

**FIG. 18**

302

330

340

**FIG. 19**

## INTERNATIONAL SEARCH REPORT

International application No.

**PCT/CN2024/070947**

### A. CLASSIFICATION OF SUBJECT MATTER

G01N21/64(2006.01)i

According to International Patent Classification (IPC) or to both national classification and IPC

### B. FIELDS SEARCHED

Minimum documentation searched (classification system followed by classification symbols)

IPC: G01N21

Documentation searched other than minimum documentation to the extent that such documents are included in the fields searched

Electronic data base consulted during the international search (name of data base and, where practicable, search terms used)

CNABS, CNTXT, CNKI, 百度学术, BAIDU SCHOLAR: 基因测序, 光, 荧光, 调制, 二值, 挡光, 阻光, 遮光, 图案, 结构光, 波长, 波段, 透镜, 屈折; VEN, USTXT, WOTXT, EPTXT: gene sequencing, light, fluorescence, modulation, two-value, shading, opacity, pattern, structural light, wavelength, lens, refraction

### C. DOCUMENTS CONSIDERED TO BE RELEVANT

| Category* | Citation of document, with indication, where appropriate, of the relevant passages | Relevant to claim No. |
|---|---|---|
| X | WO 2021179127 A1 (BGI SHENZHEN) 16 September 2021 (2021-09-16) description, page 8, paragraph 4 to page 11, paragraph 1, page 16, paragraph 5, and page 20, paragraph 5, and figures 1-5 | 1-14, 62-64 |
| Y | WO 2021179127 A1 (BGI SHENZHEN) 16 September 2021 (2021-09-16) description, page 8, paragraph 4 to page 11, paragraph 1, page 16, paragraph 5, and page 20, paragraph 5, and figures 1-5 | 17-61 |
| Y | CN 111308726 A (GENEMIND BIOSCIENCES CO., LTD.) 19 June 2020 (2020-06-19) description, paragraphs 34-36, and figure 1 | 17-61 |
| Y | JP H0627372 A (CANON KK) 04 February 1994 (1994-02-04) description, paragraph 24, and figure 1 | 50-61 |
| Y | CN 112697764 A (SHANGHAI GENESENSE TECHNOLOGY INC.) 23 April 2021 (2021-04-23) description, paragraph 90, and figures 1-9 | 17-61 |

☑ Further documents are listed in the continuation of Box C. ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **28 March 2024** | **30 March 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/070947** |

### C. DOCUMENTS CONSIDERED TO BE RELEVANT

| Category* | Citation of document, with indication, where appropriate, of the relevant passages | Relevant to claim No. |
| --- | --- | --- |
| A | CN 115452716 A (SHENZHEN SAILU MEDICAL TECHNOLOGY CO., LTD.) 09 December 2022 (2022-12-09) entire document | 1-64 |

Form PCT/ISA/210 (second sheet) (July 2022)

**INTERNATIONAL SEARCH REPORT**
Information on patent family members

International application No.

**PCT/CN2024/070947**

| Patent document cited in search report | | | Publication date (day/month/year) | Patent family member(s) | | | Publication date (day/month/year) |
|---|---|---|---|---|---|---|---|
| WO | 2021179127 | A1 | 16 September 2021 | EP | 4024031 | A1 | 06 July 2022 |
| | | | | CN | 114341622 | A | 12 April 2022 |
| | | | | HK | 40065762 | A0 | 05 August 2022 |
| CN | 111308726 | A | 19 June 2020 | CN | 209759461 | U | 10 December 2019 |
| | | | | WO | 2020119226 | A1 | 18 June 2020 |
| | | | | EP | 3896146 | A1 | 20 October 2021 |
| | | | | US | 2021373314 | A1 | 02 December 2021 |
| | | | | HK | 40062282 | A0 | 10 June 2022 |
| | | | | US | 11874453 | B2 | 16 January 2024 |
| JP | H0627372 | A | 04 February 1994 | US | 5914819 | A | 22 June 1999 |
| | | | | EP | 0572806 | A2 | 08 December 1993 |
| | | | | EP | 0572806 | A3 | 19 October 1994 |
| | | | | EP | 0572806 | B1 | 07 July 1999 |
| | | | | JP | 2979844 | B2 | 15 November 1999 |
| | | | | DE | 69325547 | D1 | 12 August 1999 |
| | | | | DE | 69325547 | T2 | 30 March 2000 |
| CN | 112697764 | A | 23 April 2021 | CN | 214310161 | U | 28 September 2021 |
| | | | | WO | 2022161169 | A1 | 04 August 2022 |
| CN | 115452716 | A | 09 December 2022 | None | | | |

Form PCT/ISA/210 (patent family annex) (July 2022)